(19) Europäisches Patentamt
European Patent Office
Office européen des brevets

(11) Publication number: **0 150 781**
**A1**

(12) # EUROPEAN PATENT APPLICATION

(21) Application number: **85100520.7**

(22) Date of filing: **18.01.85**

(51) Int. Cl.⁴: **C 07 D 499/00**, C 07 D 401/06, C 07 D 403/06, C 07 F 7/10, A 61 K 31/43 // C07F9/65, C07D239/28, C07D253/06

(30) Priority: **26.01.84 GB 8402086**

(43) Date of publication of application: **07.08.85**
**Bulletin 85/32**

(84) Designated Contracting States: **BE CH DE FR GB IT LI NL SE**

(71) Applicant: **BEECHAM GROUP PLC, Beecham House Great West Road, Brentford Middlesex TW8 9BD (GB)**

(72) Inventor: **Osborne, Neal Frederick, 9 Steeres Hill, Rusper West Sussex, RH12 4PT (GB)**

(74) Representative: **Dayneswood, Trevor, Beecham Pharmaceuticals Patent and Trade Mark Department Biosciences Research Centre Great Burgh Yew Tree Bottom Road, Epsom Surrey KT18 5XQ (GB)**

(54) **6-Alkylidene pemens, their preparation and use, and intermediates.**

(57) A compound of formula (II):

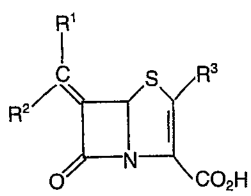

(II)

or a pharmaceutically acceptable salt or in vivo hydrolysable ester thereof, wherein one of $R^1$ or $R^2$ is hydrogen, and the other is an optionally substituted six-membered heteroaryl ring including one two or three nitrogen atoms, and $R^3$ is hydrogen or an organic group.

EP 0 150 781 A1

ACTORUM AG

B1612

NOVEL COMPOUNDS

This invention relates to β-lactam compounds and in particular to a class of 6-alkylidene penems which have β-lactamase inhibitory and antibacterial properties. The compounds are therefore useful in the treatment of antibacterial infections in humans or animals, either alone or in combination with other antibiotics.

European Patent Publication No. EP 0 041 768 A (Beecham; published 16 December 1981; corresponding to U.S. Serial No 06/257 481) discloses 6-alkylidene-2-penems of the general formula (A):

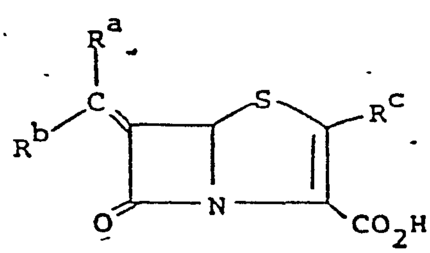

(A)

in which

each of $R^a$ and $R^b$ denotes hydrogen or an optionally substituted hydrocarbon or heterocyclic group, and

$R^c$ denotes hydrogen or an organic group.

Those compounds possess antibacterial activity and also inhibit β-lactamases and have a synergistic effect in combination with other β-lactam antibiotics.

European Patent Publication No. EP 0 120 613 A (Beecham; published 3 October 1984; corresponding to U.S. Serial No. 06/585 569) discloses a sub-group of compounds within the general formula (A) which have better activity than other compounds of the general formula (A). That sub-group consists of compounds of the general formula (B):

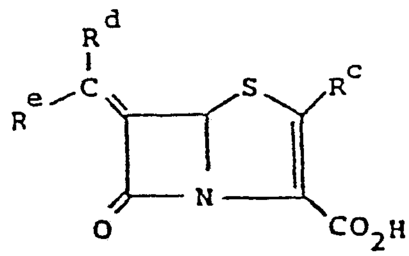

(B)

in which

$R^c$ denotes hydrogen or an organic group;

one of $R^d$ and $R^e$ denotes hydrogen, and

the other of $R^d$ and $R^e$ denotes a group of the sub-formula (C):

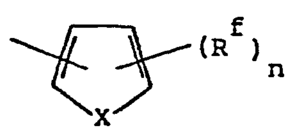

(C)

in which

$R^f$ denotes a substituent group;

X denotes an oxygen atom, a sulphur atom or an $=NR^g$ group;

$R^g$ denotes hydrogen, hydrocarbon or a nitrogen-protecting group; and

n denotes 0, 1, 2 or 3.

It has now been found that certain compounds of the general formula (A) exhibit improved β-lactamase inhibitory action and synergistic activity as compared with other compounds of that group.

According to the present invention there is provided a compound of the general formula I:

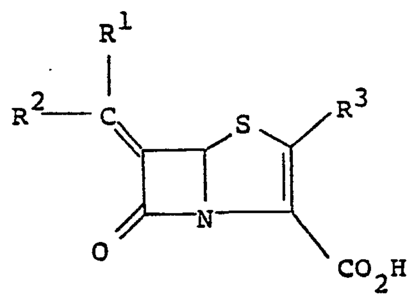

I

or a pharmaceutically acceptable salt or <u>in vivo</u> hydrolysable ester thereof

in which

one of $R^1$ and $R^2$ denotes hydrogen,

the other of $R^1$ and $R^2$ denotes an unsubstituted or substituted six-membered hetero-aromatic ring bonded through a carbon atom thereof and having from one to three nitrogen atoms as ring hetero-atoms, and

$R^3$ denotes hydrogen or an organic group.

The hetero-aromatic ring (which may also be referred to as a hetero-aryl ring) denoted by $R^1$ or $R^2$ contains six ring atoms, one, two or three of which are nitrogen atoms. The hetero-aromatic ring is bonded to the methylene carbon atom through a ring carbon atom.

The hetero-aromatic ring may be unsubstituted or may be substituted by one or more substituents, each of which may be carried on a ring carbon atom or a ring nitrogen atom, provided of course that the aromaticity of the ring is not destroyed.

Examples of suitable substituents which may be present in the hetero-aromatic ring $R^1$ or $R^2$ include $(C_{1-6})$alkanoyl, $(C_{1-6})$alkanoyloxy, heterocyclyl, amino, $(C_{1-6})$alkanoylamino, (mono or di)-$(C_{1-6})$alkylamino, hydroxy, $(C_{1-6})$alkoxy, sulpho, mercapto, $(C_{1-6})$alkylthio, $(C_{1-6})$alkylsulphinyl, $(C_{1-6})$alkyl-sulphonyl, heterocyclylthio, arylthio, sulphamoyl, carbamoyl, amidino, guanidino, nitro, halogen, carboxy, carboxy salts, carboxy esters, arylcarbonyl, and heterocyclylcarbonyl groups, and also unsubstituted or substituted $(C_{1-6})$alkyl, $(C_{2-6})$alkenyl, $(C_{2-6})$alkynyl, aryl, and aryl$(C_{1-6})$alkyl groups.

Examples of suitable optional substituents for the above-mentioned $(C_{1-6})$alkyl, $(C_{2-6})$alkenyl, $(C_{2-6})$alkynyl, aryl and aryl$(C_{1-6})$alkyl substitutents include $(C_{1-6})$alkanoyl, $(C_{1-6})$alkanoyloxy, heterocyclyl, amino, $(C_{1-6})$alkanoylamino, (mono or di)-$(C_{1-6})$alkylamino, hydroxy, $(C_{1-6})$alkylsulphinyl, $(C_{1-6})$alkylsulphonyl, heterocyclylthio, arylthio, sulphamoyl, carbamoyl, amidino, guanidino, nitro, halogen, carboxy, carboxy salts, carboxy esters, arylcarbonyl and heterocyclylcarbonyl groups.

When the hetero-aromatic ring $R^1$ or $R^2$ includes a carboxy salt or carboxy ester substituent, that substituent is suitably a pharmaceutically acceptable salt or pharmaceutically acceptable ester.

The term 'heterocyclyl' as used herein includes aromatic and non-aromatic, single and fused, rings containing up to four hetero-atoms in each ring selected from oxygen, nitrogen and sulphur, which rings may be unsubstituted or substituted by up to three groups selected from halogen, $(C_{1-6})$alkyl, $(C_{1-6})$alkoxy, halo$(C_{1-6})$alkyl, hydroxy, amino, carboxy, $(C_{1-6})$alkoxycarbonyl, $(C_{1-6})$alkoxycarbonyl$(C_{1-6})$alkyl, aryl, $(C_{1-6})$alkylthio, arylthio, mercapto and oxo groups.

The term 'aryl' as used herein includes phenyl and naphthyl, which may be unsubstituted or substituted by up to five, preferably up to three, groups selected from halogen, $(C_{1-6})$alkyl, phenyl, $(C_{1-6})$alkoxy, halo$(C_{1-6})$alkyl, hydroxy, amino, nitro, carboxy, $(C_{1-6})$alkoxycarbonyl,$(C_{1-6})$alkoxycarbonyl$(C_{1-6})$alkyl, $(C_{1-6})$alkylcarbonyloxy, $(C_{1-6})$alkylcarbonyl $(C_{1-6})$alkylthio, arylthio, and mercapto groups.

The term 'hydrocarbon' as used herein includes groups having up to 18 carbon atoms, suitably up to 10 carbon atoms, conveniently up to 6 carbon atoms. Suitable hydrocarbon groups include $(C_{1-6})$alkyl, $(C_{2-6})$alkenyl, $(C_{2-6})$alkynyl, $(C_{3-7})$cycloalkyl, $(C_{3-7})$cycloalkyl-$(C_{1-6})$alkyl, aryl, and aryl$(C_{1-6})$alkyl.

Suitably, one of $R^1$ and $R^2$ denotes hydrogen and the other of $R^1$ and $R^2$ denotes a six-membered hetero-aromatic ring of the type defined above that is

unsubstituted or is substituted by one or more $(C_{1-6})$alkyl or $(C_{1-6})$alkoxy groups, for example methyl or methoxy groups.

Suitable six-membered hetero-aromatic rings $R^1$ or $R^2$ include pyridine, pyrazine, pyrimidine, pyridazine, and triazines, each of which may be unsubstituted or substituted. (It is to be understood that, where appropriate, all isomeric forms of the above-mentioned hetero-aromatic rings are included).

Advantageously, the hetero-aromatic ring $R^1$ and $R^2$ includes at least two ring nitrogen atoms.

Examples of individual hetero-aromatic groups $R^1$ or $R^2$ include 3-pyridyl, 4-pyridyl, methoxypyridyl, pyrazinyl, 4-pyrimidinyl, 3-pyridazinyl, and dimethyltriazinyl groups.

In general formula I, $R^3$ denotes hydrogen or an organic group, which may suitably be linked through a sulphur or carbon atom. For example, $R^3$ may represent hydrogen or a group of formula $-R^4$ or $-SR^4$, where $R^4$ denotes an unsubstituted or substituted $(C_{1-10})$hydrocarbon or heterocyclyl group. Preferably, $R^3$ represents hydrogen, $(C_{1-10})$alkyl or $(C_{1-10})$alkylthio, or substituted $(C_{1-10})$alkyl or substituted $(C_{1-10})$-alkylthio, wherein the substituent may be hydroxy, $(C_{1-6})$alkoxy, $(C_{1-6})$alkanoyloxy, halogen, mercapto, $(C_{1-6})$alkylthio, heterocyclylthio, amino, (mono or di)-$(C_{1-6})$alkylamino, $(C_{1-6})$alkanoylamino, carboxy, or $(C_{1-6})$alkoxycarbonyl.

Examples of suitable organic groups $R^3$ include methyl, ethyl, propyl, methylthio, ethylthio, methylsulphinyl, ethylsulphinyl, hydroxymethyl, methoxymethyl, ethoxymethyl, acetoxymethyl, (1 or

2)-acetoxyethyl, aminomethyl, 2-aminoethyl, acetamidomethyl, 2-acetamidoethyl, carboxymethyl, 2-hydroxyethylthio, methoxymethylthio, 2-methoxyethylthio, acetoxymethylthio, 2-aminoethylthio, acetamidomethylthio, 2-acetamidoethylthio, carboxymethylthio, 2-carboxyethylthio, aryl (especially phenyl), arylthio (especially phenylthio), pyridyl, pyrimidyl, isoxazolyl, pyrimidylthio, tetrazolylthio, and pyridylthio groups. In particular, $R^3$ may be hydrogen.

Pharmaceutically acceptable *in vivo* hydrolysable esters (also referred to as 'metabolisable esters') of the compounds of the general formula I are those esters which hydrolyse in the human body to produce the parent acid or its salt. Such esters may be identified by oral or intravenous administration to a test animal, and subsequent examination of the test animal's body fluids for the presence of the compound of the formula I or a salt thereof.

In some cases, the *in vivo* hydrolysable ester moiety may constitute a link between two different active ingredient moieties, one of which is a compound according to the invention and the other of which may be another therapeutically active compound, such that on *in vivo* hydrolysis of the ester moiety, the ester link breaks to give the two separate active compounds. The linked entity may be referred to as a 'mutual pro-drug'.

Suitable *in vivo* hydrolysable ester groups include those of part-formulae (a), (b) and (c):

$$\begin{array}{c} A^1 \\ | \\ -CO_2CH\text{-}O\text{-}CO\text{-}A^2 \end{array} \qquad (a)$$

$$-CO_2\text{-}A^3\text{-}\begin{array}{c} A^4 \\ | \\ N \\ | \\ A^5 \end{array} \qquad (b)$$

$$-CO_2CH_2\text{-}OA^6 \qquad (c)$$

in which

$A^1$ denotes hydrogen, methyl, or phenyl;

$A^2$ denotes $(C_{1-6})$alkyl, $(C_{1-6})$alkoxy or phenyl; or

$A^1$ and $A^2$ together denote 1,2-phenylene, which may be unsubstituted or substituted by one or two methoxy groups;

$A^3$ denotes $(C_{1-6})$alkylene, which may be unsubstituted or substituted by a methyl or ethyl group;

each of $A^4$ and $A^5$ which may be identical or different, denotes $(C_{1-6})$alkyl; and

$A^6$ denotes $(C_{1-6})$alkyl.

Examples of suitable *in vivo* hydrolysable ester groups include acetoxymethyl, pivaloyloxymethyl, α-acetoxyethyl, α-acetoxybenzyl, α-pivaloyloxyethyl, ethoxycarbonyloxymethyl, α-ethoxycarbonyloxyethyl, dimethylaminomethyl, diethylaminomethyl, phthalidyl and dimethoxyphthalidyl groups.

Suitable pharmaceutically acceptable salts of the 3-carboxylic acid group of the compound of formula I include metal salts, e.g. aluminium salts, alkali metal salts (e.g. sodium or potassium salts), alkaline earth metal salts (e.g. calcium or magnesium salts), ammonium salts, and substituted ammonium salts, for example those with lower alkylamines (e.g.triethylamine), hydroxy-lower alkylamines (e.g. 2-hydroxyethylamine), di(2-hydroxyethyl)amine or tri(2-hydroxyethyl)amine), cycloalkylamines (e.g. dicyclohexylamine), or with procaine, and also dibenzylamine, N,N-dibenzylethylenediamine, 1-ephenamine, N-ethylpiperidine, N-benzyl-β-phenethylamine, dehydroabietylamine, N,N'-bishydroabietylethylene-diamine, bases of the pyridine type (e.g. pyridine, collidine and quinoline), and other amines which have been or can be used to form salts with penicillins.

The compounds of the general formula I and also the salts and esters thereof may exist in two optically active forms and it is to be understood that both such forms as well as racemic mixtures thereof are embraced by the present invention. It is believed that the more active form is that of structure IA:

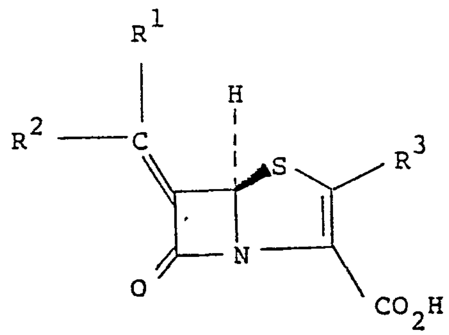

IA

in which $R^1$, $R^2$ and $R^3$ are defined as above.

Furthermore, in general formulae I and IA, it is thought to be advantageous that $R^1$ denotes the hetero-aromatic group and that $R^2$ denotes a hydrogen atom.

Examples of individual compounds according to the invention include:

(5RS) 2-methyl-(Z)-6-(3-pyridylmethylene)penem-3-carboxylic acid;

(5RS) 2-ethylthio-6-(3-pyridylmethylene)penem-3-carboxylic acid;

(5RS) 6-(3-pyridylmethylene)penem-3-carboxylic acid;

(5RS) 6-pyrazinylmethylene-penem-3-carboxylic acid;

(5RS) 6-(4-pyrimidinylmethylene)penem-3-carboxylic acid;

(5RS) 6-(4-pyridylmethylene)penem-3-carboxylic acid;

(5RS) (Z) 6-(6-methoxy-3-pyridyl)methylene-penem-3-carboxylic acid;

(5RS) (E) 6-(6-methoxy-3-pyridyl)methylene-penem-3-carboxylic acid;

(5RS) 6-(3-pyridazinyl)methylene-penem-3-carboxylic acid; and

(5RS) 6-[(5,6-dimethyl-1,2,4-triazin-3-yl)methylene] penem-3-carboxylic acid;

and pharmaceutically acceptable salts and in-vivo hydrolysable esters thereof.

A compound of the general formula I, or a salt or ester thereof, may be prepared by eliminating the elements of a compound of the general formula II:

H-X II

from a penem or penem intermediate of the general part-formula III:

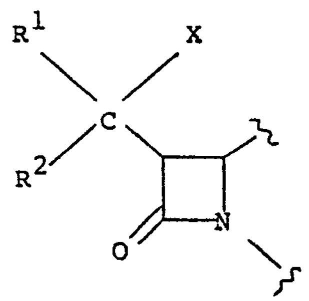

III

in which

$R^1$ and $R^2$ are defined as above, and

X denotes a hydroxy group or a leaving group,

to give a compound of the general part-formula IV:

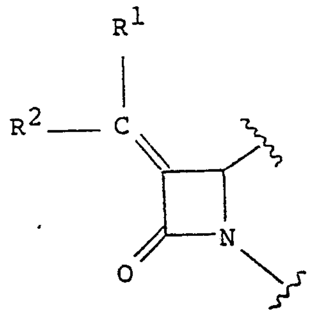

IV

in which $R^1$ and $R^2$ are defined as above,

and, if the resulting compound of the general formula IV is a penem intermediate, converting it into a penem of the general formula I or a salt or ester thereof.

The compound of the general part-formula III may suitably be a compound of the general part-formula IIIA:

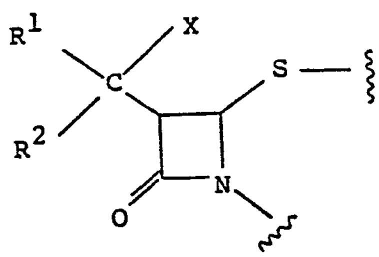

IIIA

in which $R^1$, $R^2$ and X are defined as above. More especially it may be a compound of the general formula IIIB:

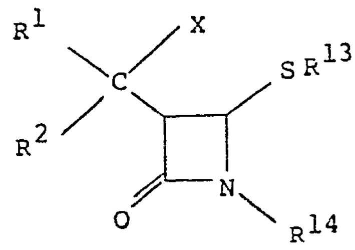

IIIB

in which

$R^1$, $R^2$ and X are defined as above,

$R^{13}$ denotes $(C_{1-6})$alkyl, aryl, aryl$(C_{1-6})$alkyl, $(C_{1-6})$alkylthio, arylthio, hetero-aromatic-thio, acyl (for example, $(C_{1-6})$alkylcarbonyl, especially acetyl), $(C_{2-6})$alkenyl (especially vinyl), or aryl$(C_{2-6})$alkenyl, all of which may optionally be substituted, and

$R^{14}$ denotes hydrogen or an N-protecting group, or

$R^{13}$ and $R^{14}$ together denote the remainder of a penem nucleus, which may be substituted and/or may optionally carry a protecting group.

In the case where $R^{13}$ and $R^{14}$ in general formula IIIB together denote the remainder of a penem nucleus, they may suitably together denote the sub-formula V:

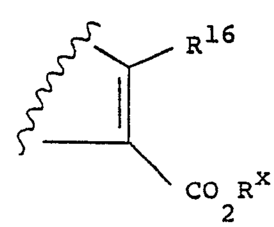

V

in which

$R^{16}$ denotes the hydrogen atom or organic group $R^3$ or a group convertible into $R^3$ during the preparation of a penem of the general formula I or salt or ester thereof, and

$R^x$ denotes hydrogen or a carboxyl-blocking group.

In that case, the penem or penem intermediate of the general part-formula III is of the general formula IIID given below.

A carboxyl-blocking group $R^x$ (also referred to as a carboxyl-protecting group) is suitably a group that can readily be removed at a later stage of the penem preparation process.

Examples of suitable carboxyl-blocking derivatives that may form the group $-CO_2R^x$ include salt, ester, and anhydride derivatives of the carboxylic acid.

The salts may be organic or inorganic and need not be pharmaceutically acceptable. Examples of suitable salt-forming groups $R^x$ include inorganic salts, for example alkali metal atoms (e.g. lithium and sodium), other metal atoms, tertiary amino groups (e.g. tri-lower-alkylamino, N-ethylpiperadino, and dimethylpiperazino groups). A preferred salt-forming group $R^x$ is the triethylamino group.

An ester-forming group $R^x$ is advantageously one that can be removed under conventional conditions. Examples of suitable ester-forming groups $R^x$ include benzyl, *p*-methoxybenzyl, 2,4,6-trimethylbenzyl, 3,5-di-t-butyl-4-hydroxy-benzyl, benzoylmethyl,

*p*-nitrobenzyl, 4-pyridylmethyl, 2,2,2-trichloroethyl, 2,2,2-tribromoethyl, allyl, acetonyl, t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, *p*-toluene-sulphonylethyl, and methoxymethyl groups, and also silyl, stannyl and phosphorus-containing groups, and oxime radicals of formula $-N=CHR^o$ in which $R^o$ denotes aryl or heterocyclyl. Furthermore, the ester-forming group $R^x$ may be an *in vivo* hydrolysable ester group including, in particular, those listed above.

The free carboxyl group may be regenerated from any of the above esters by usual methods appropriate to the particular $R^x$ group, for example, by acid-catalysed, base-catalysed or enzymically-catalysed hydrolysis, or by hydrogenation. The hydrolysis must of course be carried out under conditions in which the groups on the rest of the molecule are stable.

When it is desired to produce a compound of the general formula I in the form of a free acid or in the form of a salt, by a process according to the invention, it is generally advantageous to use a compound in which $R^x$ denotes a carboxyl-blocking group. When it is desired to produce a compound of the general formula I in the form of a pharmaceutically acceptable ester, it is generally convenient to use a compound in which $R^x$ denotes the desired ester group.

The process step according to the invention involves the elimination of the elements of a compound H-X from a penem or penem intermediate of the general part-formula III, in which X denotes a hydroxy group or a leaving group.

In the case where X denotes a hydroxy group, the compound of the formula H-X being eliminated is water and the elimination reaction is a dehydration reaction, which may suitably be carried out by treating a compound of the general part-formula III with a compound of the general formula VI:

$$R^6O_2C\text{-}N{=}N\text{-}CO_2R^7 \qquad \text{VI}$$

in which each of $R^6$ and $R^7$, which may be identical or different, denotes aryl, $(C_{1-6})$alkyl or aryl$(C_{1-6})$alkyl,

and a with compound of the general formula VII:

$$R^8(O)_a \text{---} \underset{\underset{\displaystyle (O)_cR^{10}}{|}}{P} \text{---} (O)_bR^9 \qquad \text{VII}$$

in which

each of a, b and c which may be identical or different, denotes 0 or 1, and

each of $R^8$, $R^9$ and $R^{10}$, which may be identical or different, denotes aryl, $(C_{1-6})$alkyl or aryl$(C_{1-6})$alkyl.

In the compounds of the general formula VI, $R^6$ and $R^7$ are preferably selected from methyl, ethyl, propyl, butyl, phenyl, and benzyl, the ethyl and isopropyl groups being preferred. Advantageously, $R^6$ and $R^7$ may

be identical. A preferred compound of the general formula VI is diethyl azodicarboxylate.

Preferred compounds of the general formula VII include triarylphosphines and trialkylphosphites. Preferred groups $R^8$, $R^9$ and $R^{10}$ include methyl, ethyl, n-propyl, n-butyl, benzyl, phenyl and methoxyphenyl. Advantageously, $R^8$, $R^9$ and $R^{10}$ are all identical. A preferred compound of the general formula VII is triphenylphosphine.

Advantageously, approximately two equivalents of each the compounds of the general formulae VI and VII are used per mole of the compound of the general part-formula III.

The dehydration reaction may suitably be carried out at a non-extreme temperature, for example a temperature of from $-20^oC$ to $+100^oC$. It may be convenient to begin the reaction at a depressed temperature, for example $0^oC$, and then to allow the temperature to rise to about room temperature.

The reaction may suitably be carried out in an inert aprotic organic solvent. Suitable solvents include tetrahydrofuran, dioxane, ethyl acetate, benzene, and dichloromethane.

In the cases where X, in general formula III, denotes a leaving group, which will hereinafter be referred to as $X^1$, it may suitably be a halogen atom or a group of one of the formulae

$-O-SO_2-(O)_n-R^{11}$ VIIIA

$-O-CO-(O)_n-R^{11}$ VIIIB or

$-O-PO-(OR^{12})_2$ VIIIC

in which

n denotes 0 or 1,

$R^{11}$ denotes $(C_{1-6})$alkyl, aryl or aryl$(C_{1-6})$alkyl,

and

$R^{12}$ denotes $(C_{1-6})$alkyl or aryl.

Preferred groups of formula VIIIB are those in which n denotes zero and $R^{11}$ denotes $(C_{1-6})$alkyl, especially the acetoxy group.

The elimination of the elements of a compound of the general formula II in which X denotes a leaving group $X^1$ from a compound of the general formula III,may suitably be effected by treating the compound of the general formula III with a base in an aprotic medium.

Suitable bases for that purpose include, for example, powdered inorganic bases, for example alkali metal carbonates, bicarbonates, hydroxides, and hydrides (e.g. powdered potassium carbonate), and also organic bases of low nucleophilicity, for example 1,8-diazabicyclo[5.4.0]undec-7-ene. Suitable solvents for use as the aprotic medium in this reaction include, for example, dimethylformamide, hexamethylphosphoramide, dichloromethane, and tetrahydrofuran.

The elimination may suitably be effected at a low temperature, for example a temperature of from -70°C to +70°C, advantageously from -40°C to 0°C.

The compounds of the general formula III in which X denotes a leaving group $X^1$ may suitably be prepared from the corresponding compound in which X denotes a hydroxy group by replacing the hydroxy group by a leaving group $X^1$. Alternatively, in the case of the compounds of the general formula IIID below, the leaving group $X^1$ may be introduced into the molecule at an earlier stage in the synthesis of the penem nucleus. In particular, a group $X^1$ of the formula VIIIA or VIIIB may be introduced at the beginning of, or at any stage during, the synthesis of the penem. In each case, the group $X^1$ may suitably be introduced by replacing a hydroxyl group in known manner.

The dehydration or other elimination reaction of the process according to the invention may be carried out at any suitable stage during the preparation of the penem of the general formula I or salt or ester thereof, suitably at an early stage or late stage in the manufacturing process.

Further examples of suitable leaving groups will be apparent to those skilled in the art and include sulphoxide, selenoxide and xanthate groups, which can be eliminated by known methods (see W. Carruthers, 'Some modern methods of organic synthesis', Cambridge Univ. Press 1978 (2nd edition), pages 93-103).

In particular, the dehydration or other elimination reaction may be carried out on a compound of the general formula IIIC:

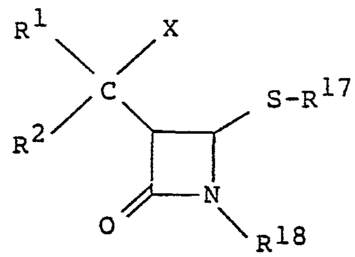

IIIC

in which

$R^1$, $R^2$ and X are defined as above,

$R^{17}$ denotes $(C_{1-6})$ alkyl, aryl, aryl$(C_{1-6})$alkyl, $(C_{1-6})$alkylthio, arylthio, hetero-aromatic-thio, acyl (for example, $(C_{1-6})$alkylcarbonyl, especially acetyl), $(C_{2-6})$alkenyl (especially vinyl), or aryl$(C_{2-6})$alkenyl, all of which may optionally be substituted, and

$R^{18}$ denotes hydrogen or an N-protecting group,

to give a compound of the general formula IVC:

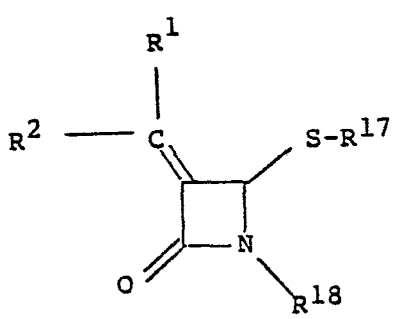

IVC

in which, $R^1$, $R^2$, $R^{17}$ and $R^{18}$ are defined as above,

which may then subsequently be converted to a penem of the general formula I or salt or ester thereof in known manner, suitably by a conventional penem preparation method.

Alternatively, the dehydration or other elimination reaction may be carried out on a compound of the general formula IIID:

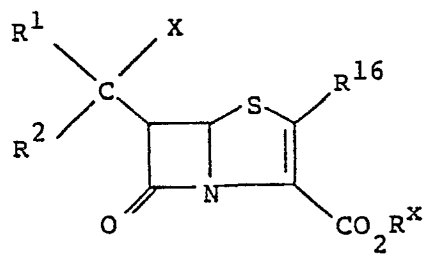

IIID

in which $R^1$, $R^2$, $R^{16}$, $R^x$ and X are defined as above, to give a compound of the general formula IVD:

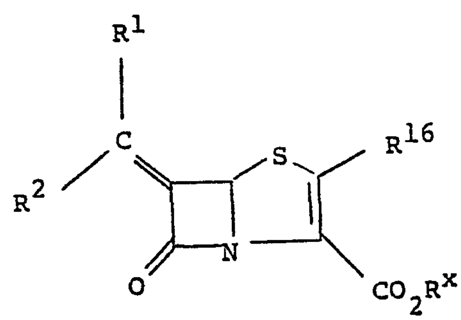

IVD

in which $R^1$, $R^2$, $R^{16}$ and $R^x$ are defined as above, and thereafter, if necessary or desired:

(a) removing any carboxyl-blocking group $R^x$,
and/or
(b) converting the group $R^{16}$ into a group or atom $R^3$,
and/or
(c) converting the product into the free acid or into a pharmaceutically acceptable salt or *in vivo* hydrolysable ester.

The conversion of a compound of the general formula IVC to the desired penem may suitably proceed *via* a compound of the general formula IVD, according to known penem preparation methods.

A compound of the general formula IIID, especially one in which X denotes a leaving group $X^1$, may conveniently be prepared from a compound of the general formula IIIC, especially one in which X denotes a leaving group $X^1$, according to known penem preparation methods.

In a compound of the general formula IIIB or IIIC, $R^{13}$ or $R^{17}$, respectively, may suitably denote a triphenylmethyl group. Examples of suitable N-protecting groups, $R^{14}$ or $R^{18}$, include silyl groups, for example t-butyldimethylsilyl groups.

In the general formula IIID and IVD, the group $R^{16}$ may be a group convertible into $R^3$ during the penem preparation process. One particular example of such a group, which may conveniently be used in the preparation of a group $R^3$ of the formula $-SR^4$ (in which $R^4$ is defined as above), is the group of the formula IX:

$$-\overset{O}{\overset{\uparrow}{S}}-R^{15} \qquad \text{IX}$$

in which $R^{15}$ denotes an organic radical different from the group $R^4$.

A sulphoxide compound of the general formula IIID or IVD in which $R^{16}$ denotes a group of the formula IX may be reacted with a thiol of the general formula XI:

$$R^4\text{-}SH \qquad XI$$

in which $R^4$ is defined as above,
or a reactive derivative thereof,
to give a compound of the general formula IIID or IVD in which $R^{16}$ denotes a group of the formula XII:

$$\text{-}S\text{-}R^4 \qquad XII$$

in which $R^4$ is defined as above.

The reaction of the sulphoxide with the thiol may be carried out as described in European Patent Publication No. EP 0 046 363A.

A sulphoxide compound of the general formula IIID or IVD in which $R^{16}$ denotes a sulphoxide group of the formula IX above may be prepared by S-oxidation of a compound of the general formula IIID or IVD, respectively, in which $R^{16}$ denotes a group of the formula $\text{-}S\text{-}R^{15}$. The S-oxidation may be effected using a mild oxidising agent, for example a perbenzoic acid, hydrogen peroxide, selenium dioxide or sodium metaperiodate. Perbenzoic acids, for example m-chloroperbenzoic acid, are preferred.

The present invention also provides a process for the preparation of a compound of the general formula I in which $R^3$ denotes a group of the formula $-SR^4$ (in which $R^4$ is defined as above), or a pharmaceutically acceptable salt or in vivo hydrolysable ester thereof, which comprises reacting a compound of the general formula XIII:

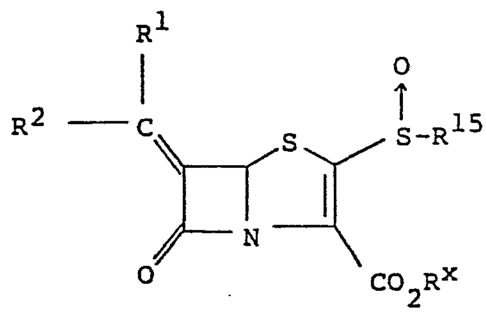

XIII

in which

$R^1$, $R^2$, $R^{15}$ and $R^x$ are defined as above,

with a thiol of the general formula XI above,

or a reactive derivative thereof;

and thereafter if necessary or desired:

(a) removing any carboxyl-blocking group $R^x$,

and/or

(b) converting the product into the free acid or into a pharmaceutically acceptable salt or *in vivo* hydrolysable ester.

The compounds of the general formulae IIIC and IVC are novel intermediates and also constitute subjects of the present invention. The compounds of the general formula IIID, in particular those in which $R^{16}$ denotes $R^3$ or a sulphoxide group of the formula IX, are also novel intermediates and form a further subject of the present invention. The compounds of the general formula IVD in which $R^{16}$ denotes a sulphoxide group of the formula IX are further novel intermediates and constitute a yet further subject of the present invention.

The compounds according to the invention have β-lactamase inhibitory and antibacterial properties, and are useful for the treatment of infections in animals, especially mammals, including humans, in particular in humans and domesticated (including farm) animals. The compounds may be used, for example, for the treatment of infections of, *inter alia*, the respiratory tract, the urinary tract, and soft tissues, especially in humans.

The compounds may be used for the treatment of infections caused by strains of, for example, *Staphylococcus aureus*, *Klebsiella aerogenes*, *Escherichia coli*, *Proteus sp.*, and *Bacteroides fragilis*. It is generally advantageous to use a compound according to the invention in admixture or conjunction with a penicillin, cephalosporin or other β-lactam antibiotic and that can often result in a synergistic effect, because of the β-lactamase inhibitory properties of the compounds according to the invention. In such cases, the compound according to the invention and the other β-lactam antibiotic can be administered separately or in the form of a single

composition containing both active ingredients as discussed in more detail below.

The compounds according to the invention are suitably provided in substantially pure form, for example at least 50% pure, advantageously at least 75% pure, preferably at least 95% pure, especially at least 98% pure, all percentages being calculated as weight/weight. An impure or less pure form of a compound according to the invention may, for example, be used in the preparation of a more pure form of the same compound or of a related compound (for example, a corresponding salt, ester or free acid) suitable for pharmaceutical use. Although the purity of any compound used as an intermediate may be less critical than that of a compound used as a final product, for example one used directly for pharmaceutical use (for example in a composition according to the invention as described below), nevertheless such an intermediate compound is advantageously provided in substantially pure form. It is generally advantageous to provide the compounds according to the invention in crystalline form.

The present invention provides a pharmaceutical composition comprising a compound according to the invention that is to say, a compound of the general formula I or a pharmaceutically acceptable salt or in vivo hydrolysable ester thereof, and a pharmaceutically acceptable carrier.

The present invention also provides a method of treating bacterial infections in animals, especially in humans and in domesticated mammals (including farm mammals), which comprises administering a compound or composition according to the invention to the animal.

Such administration may advantageously be effected in conjunction with the prior, simultaneous or subsequent administration of a pehicillin, cephalosporin or other β-lactam antibiotic.

The compositions of the invention may be in a form adapted for oral, topical or parenteral use and may be used for the treatment of infection in animals especially mammals, including humans, in particular in humans and domesticated animals (including farm animals).

The compositions of the invention may, for example, be made up in the form of tablets, capsules, creams, syrups, suspensions, solutions, reconstitutable powders, and sterile forms suitable for injection or infusion. Such compositions may contain conventional pharmaceutically acceptable materials, for example diluents, binders, colours, flavours, preservatives, and disintegrants, in accordance with conventional pharmaceutical practice in manner well understood by those skilled in the art of formulating antibiotics.

It can be particularly advantageous for the compounds according to the invention to be administered to a patient by injection or infusion. That method of administration has the advantage of rapidly resulting in high blood levels of the active ingredient compound being administered. Accordingly, in one preferred form of the composition according to the invention, a compound according to the invention is present in sterile form, including in sterile crystalline form. A further preferred form of the composition according to the invention, is one in which the composition is in injectable or infusable form.

One injectable or infusable form of the composition according to the invention is an injectable or infusable solution, which suitably comprises a solution of a compound according to the invention in a sterile pyrogen-free liquid, for example water or aqueous ethanol.

A further injectable or infusable form of the composition according to the invention is an injectable or infusable suspension, in which case the compound according to the invention is advantageously present in finely particulate form. The suspension may be an aqueous suspension in, for example, sterile water or sterile saline, which may additionally include a suspending agent, for example polyvinylpyrrolidone. Alternatively, the suspension may be an oily suspension in a pharmaceutically acceptable oil suspending agent, for example arachis oil.

A composition according to the invention may be in unit dosage form, for example unit dosage form for oral administration, topical administration, or parenteral administration (including administration by injection or infusion).

A composition according to the invention may comprise a compound according to the invention as the sole active ingredient or therapeutic agent, or it may also comprise one or more additional active ingredients or therapeutic agents, for example a penicillin, cephalosporin or other β-lactam antibiotic, or pro-drug thereof. A composition comprising a compound according to the invention and another active ingredient or therapeutic agent, especially a penicillin, cephalosporin or other β-lactam antibiotic, or pro-drug thereof, can show enhanced effectiveness, and in particular can show a synergistic effect.

Penicillins, cephalosporins and other β-lactam antibiotics suitable for co-administration with the compounds according to the invention - whether by separate administration or by inclusion in the compositions according to the invention - include both those known to show instability to or to be otherwise susceptible to β-lactamases and also those known to have a degree of resistance to β-lactamases.

Examples of penicillins suitable for co-administration with the compounds according to the invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, sulbenicillin, piperacillin, and other known penicillins. The penicillins may be used in the form of pro-drugs thereof, for example as *in vivo* hydrolysable esters,for example the acetoxymethyl, pivaloyloxymethyl, α-ethoxycarbonyloxyethyl and phthalidyl esters of ampicillin, benzylpenicillin and amoxycillin; as aldehyde or ketone adducts of penicillins containing a 6-α-aminoacetamido side chain (for example hetacillin, metampicillin and analogous derivatives of amoxycillin); and as α-esters of carbenicillin and ticarcillin, for example the phenyl and indanyl α-esters.

Examples of cephalosporins that may be co-administered with the compounds according to the invention include, cefatrizine, cephaloridine, cephalothin, cefazolin, cephalexin, cephacetrile, cephapirin, cephamandole nafate, cephradine, 4-hydroxycephalexin, cephaloglycin, cefoperazone, cefsulodin, ceftazidime, cefuroxime, cefmetazole, cefotaxime, ceftriaxone, and other known

cephalosporins, all of which may be used in the form of pro-drugs thereof.

Examples of β-lactam antibiotics other than penicillins and cephalosporins that may be co-administered with the compounds according to the invention include aztreonam, latamoxef (Moxalactam - Trade Mark), and other known β-lactam antibiotics, all of which may be used in the form of pro-drugs thereof.

In the compositions according to the invention, the compound according to the invention and the penicillin, cephalosporin or other β-lactam antibiotic may be linked by means of an in vivo hydrolysable ester group, in the form of a mutual pro-drug.

Some penicillins and cephalosporins that may be included in the compositions according to the invention may not be suitable for oral administration, in which case the composition will be in a form suitable for parenteral or topical administration.

Particularly suitable penicillins for co-administration with the compounds according to the invention include ampicillin, amoxycillin, carbenicillin, piperacillin, azlocillin, mezlocillin, and ticarcillin. Such penicillins may be used in the form of their pharmaceutically acceptable salts, for example their sodium salts. Alternatively, ampicillin or amoxycillin may be used in the form of fine particles of the zwitterionic form (generally as ampicillin trihydrate or amoxycillin trihydrate) for use in an injectable or infusable suspension, for example, in the manner hereinbefore described in relation to the compounds according to the invention. Amoxycillin, for example in the form of its sodium salt or the trihydrate, is particularly preferred for use in synergistic compositions according to the invention.

Particularly suitable cephalosporins for co-administration with the compounds according to the invention include cephaloridine, cefoperazone and cefazolin, which may be used in the form of their pharmaceutically acceptable salts, for example their sodium salts.

A compound according to the invention may be administered to the patient in an antibacterially effective amount or, when a compound according to the invention is being used in conjunction with a penicillin, cephalosporin, or other β-lactam antibiotic, it may be used in a synergistically effective amount.

The compounds according to the invention may suitably be administered to the patient at a daily dosage of from 0.7 to 50 mg/kg of body weight. For an adult human (of approximately 70 kg body weight), from 50 to 3000 mg, preferably from 100 to 1000 mg, of a compound according to the invention may be adminstered daily, suitably in from 1 to 6, preferably from 2 to 4, separate doses. Higher or lower dosages may, however, be used in accordance with clinical practice.

When the compositions according to the invention are presented in unit dosage form, each unit dose may suitably comprise from 25 to 1000 mg, preferably from 50 to 500 mg, of a compound according to the invention. Each unit dose may, for example, be 62.5, 100, 125, 150, 200 or 250 mg of a compound according to the invention.

When the compounds according to the invention are co-administered with a penicillin, cephalosporin or

other β-lactam antibiotic, the ratio of the amount of the compound according to the invention to the amount of the other β-lactam antibiotic may vary within a wide range. The said ratio may, for example, be from 100:1 to 1:100; more particularly, it may, for example, be from 2:1 to 1:30.

The amount of penicillin or cephalosporin or other β-lactam antibiotic in a synergistic composition according to the invention will normally be approximately similar to the amount in which it is conventionally used *per se*, for example from about 50 mg, advantageously from about 62.5 mg, to about 3000 mg per unit dose, more usually about 125, 250, 500 or 1000 mg per unit dose.

An example of a particularly suitable composition according to the invention is one comprising from 150 to 1000 mg, preferably from 200 to 700 mg, of amoxycillin or ampicillin or a pro-drug thereof, in admixture or conjunction with from 5 to 500 mg, preferably from 20 to 250 mg, of a compound according to the invention, per unit dose. In such a composition, the amoxycillin may suitably be in the form of its trihydrate or sodium salt; the ampicillin may suitably be in the form of ampicillin trihydrate, ampicillin anhydrate, sodium ampicillin, hetacillin, pivampicillin hydrochloride, bacampicillin hydrochloride or talampicillin hydrochloride; and the compound according to the invention may most suitably be in crystalline form. Such composition may be in a form suitable for oral or parenteral use, except when it comprises an *in vivo* hydrolysable ester of ampicillin or amoxycillin in which case the composition should not normally be intended for parenteral administration.

A further example of a particularly suitable composition according to the invention is one comprising from 200 to 2000 mg of carbenicillin or ticarcillin or a pro-drug thereof, in admixture or conjunction with from 5 to 500 mg, preferably from 25 to 250 mg, of a compound according to the invention, per unit dose. In such a composition, the carbenicillin or ticarcillin may most suitably be in the form of its di-sodium salt, and the compound according to the invention may most suitably be in crystaline form. When the composition contains the carbenicillin or ticarcillin in the form of a di-salt, it is most suitably presented in a form suitable for parenteral administration.

No toxicological effects are indicated when the compounds according to the invention are administered within the above-mentioned dosage ranges.

The following examples illustrate the invention. Unless otherwise stated, all temperatures are given in degrees Celsius and all percentages are calculated by weight. The term 'Biogel' used in the examples is a Trade Mark.

Preparation 1(a)

(3RS, 4SR) 1-t-Butyldimethylsilyl-3-[hydroxy (3-pyridyl)methyl]-4-tritylthioazetidin-2-one

A solution of n- butyl lithium (1.9M in hexane, 12.6 ml) was added to a stirred solution of diisopropylamine (3.35 ml) in dry tetrahydrofuran (THF) (100 ml) at -30°C under dry argon. After stirring at -30°C for 10 minutes the mixture was cooled to -76°C and treated, dropwise over 5 minutes, with a solution of 1-t-butyldimethylsilyl-4-tritylthioazetidin-2-one (1) (9.18g) (Bristol-Myers Patent GB 2042515A) in dry THF (50 ml). After a further 15 minutes at -76°C the stirred mixture was treated, dropwise over 10 minutes, with a solution of freshly distilled 3-pyridinecarboxaldehyde (2.78g) in dry THF (10 ml). After 20 minutes at -76°C the stirred mixture was treated with saturated aqueous ammonium chloride solution (50 ml) and allowed to attain room temperature. The mixture was diluted with ethyl acetate (500 ml) and washed with brine (3 x 50 ml). The dried ($MgSO_4$) organic layer was evaporated and the residue chromatographed on silica gel eluting with ethyl acetate/hexane mixtures to give the title compound (2) (8.14g) as a solid, mp 108 - 110°C (fine needles ex ethyl acetate/hexane); νmax ($CHCl_3$) 3600-3100, 1735 $cm^{-1}$; δppm ($CDCl_3$) -0.03 and -0.02 (6H, eachs), 0.79 (9H, s), 2.5-2.9 (1H, broad signal), 3.78 (1H, dd, J 1.7 and 6.2Hz), 3.94 (1H, d, J 1.7Hz), 4.07 (1H, d, J 6.2Hz), 7.1-7.5 (17H, m), 7.99-8.02 (1H, m), 8.46-8.50 (1H, m); (Found: C, 72.5; H, 7.1;, N, 4.7; S, 5.5. $C_{34}H_{38}N_2O_2$ S Si requires C, 72.1; H, 6.7; N, 4.9; S, 5.7%).

Preparation 1(b)

(3RS, 4SR) 3-[Acetoxy (3-pyridyl)methyl]-1-t-butyldimethylsilyl-4-tritylthioazetidin-2-one

A solution of acetic anhydride (1.62 ml) in dry dichloromethane (5ml) was added, dropwise over 5 minutes, to a stirred, ice bath cooled, mixture of the trans-azetidinone (2) (8.07g) from Preparation 1(a), 4-dimethylaminopyridine (157 mg) and triethylamine (2.38 ml) in dry dichloromethane (200 ml). After stirring at room temperature for 2 hours the mixture was washed with 5% citric acid (20 ml), brine (20 ml), saturated $NaHCO_3$ (20 ml) and brine (3 x 20 ml). The dried ($MgSO_4$) organic layer was evaporated and the residue chromatographed on silica gel eluting with ethyl acetate/hexane mixtures to give the title acetate (3) (7.52g) as an amorphous solid, νmax ($CHCl_3$) 1745 $cm^{-1}$; δppm ($CDCl_3$) -0.09 and -0.08 (6H, each s), 0.75 (9H, s), 2.00 (3H, s), 3.83 (1H, d, J 2.0Hz), 3.89 (1H, dd, J 2.0 and 5.9Hz), 5.07 (1H, d, J 5.9Hz), 7.15-7.55 (17H, m), 8.03-8.07 (1H, m), 8.49-8.54 (1H, m).

Preparation 1(c)

(3RS, 4SR) 3-[Acetoxy (3-pyridyl)methyl]-4-tritylthioazetidin-2-one

A solution of anhydrous potassium fluoride (786 mg) in methanol (20 ml) was added, dropwise over 15 minutes, to a stirred solution of the acetate (3) (7.50g) from Preparation 1(b) in methanol (200 ml) at -20°C. After stirring at -20°C for 1 hour the mixture was poured into water (600 ml) and extracted with dichloromethane (3 x 200 ml). The combined organic layers were washed with brine (2 x 100 ml), dried ($MgSO_4$), and evaporated to give a crude gum. Trituration of the crude gum with ether gave the title azetidinone (4) (5.75g) as a solid, mp 219-220°C (prisms ex chloroform/ether), νmax ($CHCl_3$) 3380, 1770 $cm^{-1}$; δ ppm ($CDCl_3$) 2.17 (3H, s), 3.61 (1H, ddd, J 6.2, 2,8 and 1.0Hz), 4.28 (1H, slightly broadened s), 4.40 (1H, d, J 2.8Hz), 6.13 (1H, d, J 6.2Hz), 7.20-7.42 (16H, m), 7.60-7.66 (1H, m), 8.54-8.67 (2H, m). (Found: C, 72.9; H, 5.3; N, 5.4; S, 6.9; $C_{30}H_{26}N_2O_3S$ requires C, 72.8; H, 5.3; N, 5.7; S, 6.5%).

Preparation 1(d)

(3RS, 4SR) 3-[Acetoxy (3-pyridyl)methyl]-1-(1-p-nitrobenzyloxycarbonyl-1-triphenylphosphoranylidenemethyl)-4-tritylthioazetidin-2-one

The azetidinone (4) (5.50g) from Preparation 1(c) and p-nitrobenzyl glyoxylate monohydrate (2.78g) were heated in refluxing benzene (100 ml) with provision for azeotropic removal of water (Dean and Stark apparatus containing molecular sieves 4A) for 1 hour. The mixture was cooled to room temperature and treated with triethylamine (112 mg). After stirring at room temperature for 30 minutes the mixture was evaporated to give a crude hydroxyester (5) as an amorphous solid, νmax ($CHCl_3$) 3600-3100, 1770 br $cm^{-1}$. A solution of the crude hydroxyester (5) in dry THF (100 ml) was cooled to $-10^{o}C$ and treated with 2,6-lutidine (1.94 ml) followed by a solution of thionyl chloride (1.21 ml) in dry THF (5 ml) dropwise over 10 minutes. After stirring at $-10^{o}C$ for a further 10 minutes the mixture was filtered and evaporated. The residue was re-evaporated from dry toluene (2 x 10 ml) to give a crude chloroester (6) as an amorphous solid, νmax ($CHCl_3$) 1780 br.$cm^{-1}$. A mixture of the crude chloroester (6), triphenylphosphine (11.7g) and 2,6-lutidine (1.55 ml) were heated in dry dioxan (150 ml) at $100^{o}C$ under dry argon for 12 hours. The mixture was diluted with ethyl acetate (500 ml) and was washed with 1N. hydrochloric acid (20 ml), brine (20 ml), saturated $NaHCO_3$ (20 ml) and brine (3 x 20 ml). The dried ($MgSO_4$) organic layer was evaporated and the residue chromatographed on silica gel eluting with ethyl acetate/hexane mixtures to give the title phosphorane (7) (7.79g), νmax ($CHCl_3$) 1750, 1620, 1605 sh. $cm^{-1}$.

Preparation 1(e)

(3RS, 4SR) Silver 3-[Acetoxy (3-pyridyl)methyl]-1-(1-p-nitrobenzyloxycarbonyl-1-triphenylphosphoranylidenemethyl)azetidin-2-one-4-thiolate

The phosphorane (7) (200 mg) from Preparation 1(d) was dissolved in methanol (2 ml) and treated with pyridine (22 mg) and silver nitrate (1.8 ml of a 0.15M solution in methanol). After 30 minutes at room temperature the stirred mixture was cooled in an ice bath for 10 minutes and filtered. The residue was washed with cold methanol (1 ml) and dry ether (3 x 1 ml) and dried under vacuum to give the title silver salt (8) (152 mg) as an off-white amorphous solid, ν max (Nujol) 1745, 1600 $cm^{-1}$.

Preparation 1(f)

(3RS, 4SR) 3-[Acetoxy (3-pyridyl)methyl]-4-acetylthio-1-(1-p-nitrobenzyloxycarbonyl-1-triphenylphosphoranylidenemethyl)azetidin-2-one

A solution of the silver salt (8) (150 mg) from Preparation 1(e) was dissolved in dry dichloromethane, cooled in an ice bath, and treated with pyridine (51 mg) and acetyl chloride (44 mg). The mixture was stirred at ice bath temperature for 15 minutes, filtered, and the residue washed with ethyl acetate (10 ml). The combined filtrates were washed with 5% citric (1 ml), brine (1 ml), saturated $NaHCO_3$ (1 ml) and brine (3 x 1 ml). The dried ($MgSO_4$) organic layer was evaporated and the residue chromatographed on silica gel eluting with ethyl acetate/hexane mixtures to give the title phosphorane (9) (95 mg) as an amorphous solid, νmax ($CHCl_3$) 1760, 1695, 1620, 1605 sh. $cm^{-1}$.

Preparation 1(g)

(5RS, 6RS) p-Nitrobenzyl 6-[Acetoxy (3-pyridyl)methyl]-2-methylpenem-3-carboxylate

The phosphorane (9) (90 mg) from Preparation 1(f) was heated in refluxing toluene (45 ml) under dry argon for 5 hours. The mixture was evaporated and the residue chromatographed on silica gel eluting with ethyl acetate/hexane mixtures to give after crystallisation from ethyl acetate/hexane the title penem (10) (39 mg), mp 136-137°C (needles); λmax (EtOH) 261 (Em 15285) and 310 nm (8860); νmax ($CHCl_3$) 1790, 1740, 1710 $cm^{-1}$; δppm ($CDCl_3$) 2.17 (3H, s), 2.39 (3H, s), 4.21 (1H, dd, J 6.5, and 1.7Hz), 5.25 and 5.41 (2H, ABq, J13Hz), 5.61 (1H, d, J 1.7Hz), 6.22 (1H, d, J 6.5Hz), 7.31-7.37 (1H, m), 7.60 (2H, d, J 9Hz), 7.67-7.73 (1H, m), 8.25 (2H, d, J 9Hz), 8.66 (2H, broad s). (Found: C, 56.4; H, 4.2; N, 8.8; S, 6.9; $M^+$ 469.0957. $C_{22}H_{19}N_3O_7S$ requires C, 56.3; H, 4.1; N, 9.0; S, 6.8%; M, 469.0943).

Example 1(a)

(5RS) p-Nitrobenzyl 2-Methyl-6-(3-pyridylmethylene) penem-3-carboxylate

A solution of the penem (10) (40 mg) from Preparation 1(g) in dry dichloromethane (2 ml) was cooled to -40°C and treated with 1,8-diazabicyclo [5.4.0] undec-7-ene (DBU) (19 mg). After stirring at -40°C for 30 minutes the mixture was diluted with ethyl acetate (10 ml) and washed with 5% citric acid (1 ml), brine (1 ml), saturated $NaHCO_3$ (1 ml) and brine (3 x 1 ml). The dried ($MgSO_4$) organic layer was evaporated and the residue chromatographed on silica gel eluting with dichloromethane/ethyl acetate mixtures to give the title penem (11) (27 mg), a 4:1 mixture of (Z) and (E)-isomers, as an amorphous solid, δppm ($CDCl_3$) 2.40 (3H,s), 5.23 and 5.49 (2H, ABq, J 14Hz), 6.22 (0.2H, s), 6.42 (0.8H, s), 6.61 (0.2H, s), 7.12 (0.8H, s), 7.25-7.60 (2H, m), 7.65 (2H, d, J 9Hz), 8.24 (2H, d, J 9Hz), 8.61 (2H, broads). The mixture was crystallised twice from ethyl acetate/hexane to give the pure (Z)-isomer, mp 181-184°C (pale yellow needles) λmax (EtOH) 270 (Em 29820) and approximately 291 nm (inflection); νmax ($CHCl_3$) 1780, 1710 $cm^{-1}$; δppm ($CDCl_3$) 2.40 (3H, s), 5.23 and 5.49 (2H, ABq, J14Hz), 6.42 (1H, s), 7.12 (1H, s) 7.27-7.60 (2H, m), 7.64 (2H, d, J 9Hz), 8.22 (2H, d, J 9Hz), 8.55-8.70 (2H, m). (Found: C, 58.9; H, 3.9; N, 10.3; $M^+$, 409.0708. $C_{20}H_{15}N_3O_5S$ requires C, 58.7; H, 3.7; N, 10.3; M, 409.0730).

Example 1(b)

(5RS) Sodium 2-Methyl-(Z)-6-(3-pyridylmethylene) penem-3-carboxylate

The penem ester (11) (Z-isomer (30 mg) from Example 1(a) was dissolved in a mixture of dioxan (8 ml) and water (2 ml) and was hydrogenated over 5% palladium/charcoal catalyst (45 mg) at S.T.P. for 40 minutes. Further catalyst (30 mg) was added and the hydrogenation was continued for 40 minutes. A 1% sodium bicarbonate solution was added and the mixture was filtered through Kieselguhr the residue being washed with a little aqueous dioxan. The combined filtrates were evaporated and the residue was chromatographed on Biogel P2 eluting with water. The appropriate fractions were evaporated and the residue re-evaporated from ethanol (1 ml) and dry toluene to give, after trituration with dry ether, the title sodium salt (12) (7.5 mg) as an orange yellow amorphous solid, λmax ($H_2O$) 268 (Em 18,900) and 370 nm (1890); δppm ($D_2O$) 2.26 (3H, s), 6.58 (1H, s), 7.21 (1H, s), 7.46-7.56 (1H, m), 7.81-7.87 (1H, m), 8.42-8.65 (2H, broad signal).

Preparation 2(a)

(3RS, 4SR) 3-[Acetoxy (3-pyridyl)methyl]-4-ethylthiothiocarbonylthio-1-(1-p-nitrobenzyloxycarbonyl-1-triphenylphosphoranylidenemethyl) azetidin-2-one

A stirred, ice bath cooled, solution of the silver salt (8) (540 mg) from Preparation 1(e) in dry dichloromethane (10 ml) was treated with pyridine (157 mg) and ethyl dithiochloroformate (187 mg). The ice bath was removed and the mixture was stirred for 1 hour. The mixture was filtered through Kieselguhr and the residue was washed with dichloromethane. The combined filtrates were concentrated diluted with ethyl acetate (50 ml), and washed with 5% citric acid (10 ml), brine (10 ml), saturated sodium bicarbonate (10 ml) and brine (3 x 10 ml). The dried ($MgSO_4$) organic layer was evaporated and the residue chromatographed on silica gel eluting with ethyl acetate/hexane mixtures to give the title phosphorane (13) (320 mg) as a yellow amorphous solid, $\nu$max ($CHCl_3$) 1760, 1620 $cm^{-1}$.

Preparation 2(b)

(5RS, 6SR) and (5RS, 6RS) p-Nitrobenzyl 6-[Acetoxy (3-pyridyl)methyl]-2- ethylthiopenem-3-carboxylate

The phosphorane (13) (150 mg) from Preparatino 2(a) was heated in refluxing xylene (75 ml) under argon for 8 hours. The mixture was cooled, evaporated and the residue chromatographed on silica gel eluting with ethyl acetate/hexane mixtures to give a 3:1 mixture of the title trans and cis-penems (14) (30 mg) as an amorphous solid, λmax (EtOH) 261 (Em 17,680) and 336 nm (9,270); νmax ($CHCl_3$) 1795, 1745, 1690 $cm^{-1}$; δppm ($CDCl_3$) 1.1-1.5 (3H, m), 2.07 and 2.10 (3H, each s), 2.8-3.1 (2H, m), 4.20 (0.75H, dd, J 1.4 and 6.6Hz), 4.44 (0.25H, dd, J 3.6 and 10.3Hz), 5.08-5.50 (2H, m), 5.66 (0.75H, d, J 1.4Hz), 5.82 (0.25H, d, J 3.6Hz), 6.14 (d, J 10.3Hz) and 6.19 (d, J 6.6Hz) together 1H, 7.30-7.85 (4H, m), 8.1-8.3 (2H, m), 8.6-8.8 br (2H, m).

Example 2(a)

(5RS) p-Nitrobenzyl 2-Ethylthio-6-(3-pyridylmethylene)penem-3-carboxylate

The penem (14) (a mixture of trans and cis isomers) (60 mg) from Preparation 2(b) was treated with DBU (27 mg) as for Example 1(a) to give the title penem (15) (44 mg), a 2:1 mixture of (Z) and (E)-isomers, as an orange yellow solid, λmax (EtOH) 267 (Em 30,205) and 325 nm (inflection); νmax ($CHCl_3$) 1780, 1690 $cm^{-1}$; δppm ($CDCl_3$) 1.38 (3H, t, J 7Hz), 2.75-3.20 (2H, m), 5.24 and 5.51 (2H, ABq, J14 Hz), 6.30 ($\frac{1}{3}$H, s), 6.51 ($\frac{2}{3}$H, slightly broadened s), 6.65 ($\frac{1}{3}$H, slightly broadened s), 7.14 ($\frac{2}{3}$H, slightly broadened s), 7.25-7.70 (m) and 7.65 (d, J 8.5Hz), together 4H, 8.22 (2H, d, J 8.5Hz), 8.5 - 8.9 br(2H, m). (Found: $M^+$, 455.0620. $C_{21}H_{17}N_3O_5S_2$ requires M, 455.0609).

Example 2(b)

(5RS) Sodium 2-Ethylthio-6-(3-pyridylmethylene)penem-3-carboxylate

The penem ester (15) (a 2:1 mixture of (Z) and (E) isomers) (40 mg) from Example 2(a) was hydrogenated as for Example 1(b) to give the title sodium salt (16) (7.3 mg), a 3:1 mixture of (Z) and (E) isomers, as an orange-yellow amorphous solid, λmax ($H_2O$) 272 (Em 18230) and 390 nm (2820); δppm ($D_2O$) 1.27-1.34 (3H, m), 2.76-3.06 (2H, m), 6.35 (0.25H, s), 6.66 (0.75H, s), 6.89 (0.25H, s), 7.22 (0.75H, s,), 7.50-7.62 (1H, m), 7.84-7.90 (1H, m), 8.5-8.6 (2H, m).

Preparation 3(a)

(5RS, 6SR) p-Nitrobenzyl 6-[Acetoxy (3-pyridyl)methyl] penem-3-carboxylate

A stirred, ice bath cooled, solution of the silver thiolate (8) (200 mg) from Preparation 1(e) was treated with acetic-formic anhydride (0.30 ml) and triethylamine hydrochloride (68 mg). The ice bath was removed and the mixture was stirred for 15 minutes. The mixture was filtered through Kieselguhr and the residue was washed with ethyl acetate (5 ml). The combined filtrates were diluted with ethyl acetate (25 ml) and washed with water (5 ml), saturated $NaHCO_3$ solution (5 ml) and brine (3 x 5 ml). The dried ($MgSO_4$) organic layer was diluted to approximately 50 ml with ethyl acetate and heated at 50$^{o}$C under argon for 30 minutes. The mixture was evaporated and the residue chromatographed on silica gel eluting with ethyl acetate/chloroform mixtures to give the title penem (17) (41 mg) as an amorphous solid, $\lambda$max (EtOH) 260 (Em 14620) and 314 nm (7575); $\nu$max ($CHCl_3$) 1800, 1745 sh, 1720 cm$^{-1}$; $\delta$ppm ($CDCl_3$) 2.12 (3H, s), 4.29 (1H, ddd, J 6.3, 1.9 and approximately 1.0Hz), 5.21 and 5.41 (2H, ABq, J 14Hz), 5.78 (1H, d, J 1.9Hz), 6.18 (1H, d, J 6.3Hz), 7.2-7.8 (5H, m) 8.20 (2H, d, J 8Hz), 8.5-8.7 (2H, m). (Found: MH]$^+$, 456.0826. $C_{21}H_{18}N_3O_7S$ requires 456.0865).

Example 3(a)

(5RS) p-Nitrobenzyl 6-(3-pyridylmethylene)penem-3-carboxylate

The penem (17) (100 mg) from Preparation 3(a) was treated with DBU (50 mg) as for Example 1(a) to give the title penem (18) (69 mg), a 4:1 mixture of (Z) and (E)-isomers, as a yellow solid, λmax (EtOH) 270 (Em 27665) and 292 nm (21725); νmax ($CHCl_3$) 1785, 1720 $cm^{-1}$; δ ppm ($CDCl_3$) 5.31 and 5.48 (2H, ABq, J14Hz), 6.47 (0.2H, s), 6.66 (0.8H, s), 6.69 (0.2H, s), 7.20 (0.8H, s), 7.36-7.48 (2H, m), 7.60-7.67 (3H, m), 8.26 (2H, d, J8Hz), 8.61-8.90 (2H, m). (Found: $M^+$, 395.0584. $C_{19}H_{13}N_3O_5S$ requires M, 395.0576).

Example 3(b)

(5RS) Sodium 6-(3-Pyridylmethylene)penem-3-carboxylate

The penem ester (18) (a mixture of Z and E-isomers) (60 mg) from Example 3(a) was hydrogenated as for Example 1(b) to give the title sodium salt (19) (15 mg), an approximately 6:1 mixture of (Z) and (E) -isomers, as a yellow amorphous solid, λmax (EtOH) 275 (Em 15,500), 292 (15065) and 370 nm (1710); δppm ($D_2O$) 6.48 (1/7H, s), 6.79 (6/7H, s), 6.88 (1/7H, s), 7.06 (6/7H, s), 7.15 (1/7H, s), 7.24 (6/7H, s), 7.48-7.56 (1H, m), 7.82-7.88 (1H, m), 8.46-8.60 (2H, m).

Preparation 4a

(3RS, 4SR) N-t-Butyldimethylsily-3-pyrazinylcarbonyl-4-tritylthio-azetidin-2-one

n-Butyl lithium in n-hexane (1.64M; 14.6 ml) was added to a solution of diisopropylamine (3.22 ml) in dry tetrahydrofuran (THF) (80 ml) at -60° in an atmosphere of argon. After 30 minutes a solution of azetidinone (1) (9.2g) in THF (30 ml) was added dropwise with stirring.

The resulting pink solution was maintained at -60° for 30 minutes and a solution of methyl pyrazine-carboxylate (6g; 2.2eq) in THF (20 ml) was added in one portion with stirring. After 15 minutes at -60° the dark solution was diluted with saturated aqueous ammonium chloride and brine. The aqueous mixture was extracted with ethyl acetate (200 ml) and the extract was washed with brine and dried ($MgSO_4$). Evaporation gave the crude product as a gum.

Chromatography (silica gel eluted with ethyl acetate/n-hexane) gave the title compound as a white solid (8.44g).

When recrystallized from ethyl acetate/n-hexane the azetidinone-Ketone (20) had m.p. 154-156°; ir νmax ($CHCl_3$) 1745, 1690 $cm^{-1}$; δ ($CDCL_3$) 0.99 (9H, s); 4.98 (1H, d, J=2Hz); 5.05 (1H, d, J=2Hz); 7.0-7.5 (15H, m); 8.6-8.75 (2H, m); 9.0 (1H, d, J=ca. 1Hz); signal for $Me_2Si$ not observed.

Preparation 4(b)

(3RS, 4SR) N-t-Butyldimethylsilyl-3-[hydroxy(pyrazinyl)methyl]-4-tritylthio-azetidin-2-one

The azetidinone-Ketone (20) from Preparation 4a (8.2g) was dissolved in dioxan (120 ml) containing pH7 phosphate buffer (10 ml) and cooled in an ice bath. Sodium borohydride (600 mg) was added in portions with stirring over a period of 5 minutes.

The cooling bath was removed and the solution allowed to attain room temperature. After 40 minutes the solution was diluted with brine and with ethyl acetate (ca 200 ml). The organic phase was separated, washed with brine, dried ($MgSO_4$) and evaporated. Chromatography (silica/ethyl acetate-n hexane) of the residue gave two isomers:-

isomer I (21), 1.73g, crystallized from ethyl acetate-n-hexane had m.p. 163-165$^o$; νmax ($CHCl_3$) 3300-3500, 1745 $cm^{-1}$; ∂($CDCl_3$) 0.93 (9H, s); 3.38 (1H, dd, J=2.7, 1.9Hz); 3.75 (1H, dd, J=6, 2.7Hz) collapses to d, J=2.7 with $D_2O$; 4.06 (1H, d, J=6, exch. $D_2O$); 4.68 (1H, d, J=1.9Hz); 7.1-7.7 (ca. 15H, m); 8.25-8.5 (3H, m).

isomer II (22), 4.76g, crystallized form ethyl acetate-n-hexane had m.p. 79-81$^o$; νmax ($CHCl_3$) 3200-3500, 1735 $cm^{-1}$; ∂($CDCl_3$) 0.8 (9H, s); 3.05 (1H, d, J=9Hz, exch. $D_2O$); 3.81 (1H, dd, J=6.3, 1.8Hz); 4.21 (1H, d, J=1.8Hz); 4.37 (1H, dd, J=9, 6.3Hz) collapses to d, J= 6.3Hz with $D_2O$; 7.15-7.5 (ca 15H, m); 8.3-8.5 (3H, m).

Preparation 4(c)

(3RS, 4SR) N-t-Butyldimethylsilyl-3-[acetoxy(pyrazinyl)methyl]4-tritylthioazetidin-2-one

The azetidinone-alcohol (22) (Preparation 4b, isomer II, 4.54g) was dissolved in dry dichloromethane (65 ml) containing triethylamine (2.05 mls; 1.5 eq) and 4-dimethylaminopyridine (40 mg). The solution was cooled in an ice bath and acetic anhydride (3.7 ml; 5eq) was added.

The cooling bath was removed and the reaction mixture allowed to stand at room temperature for 1 hour. The solution was then washed with dilute hydrochloric acid, with aqueous sodium hydrogen carbonate and with brine. Drying ($MgSO_4$) and evaporation gave a crude product which was chromatographed (silica gel-ethyl acetate-n-hexane).

The acetate (23), 4.4g, had an indistinct melting point, 142-150$^o$, when recrystallized form methanol; νmax ($CHCl_3$) 1745 $cm^{-1}$; ∂($CDCl_3$) 0.74 (9H, s); 1.98 (3H, s); 3.97 (1H, dd, J=6, 2Hz); 4.34 (1H, d, J=2Hz); 5.28 (1H, d, J=6Hz); 7.1-7.6 (ca 15H, m); 8.25-8.35 (3H, m).

Preparation 4(d)

(3RS, 4SR) 3-[Acetoxy(pyrazinyl)methyl]-4-tritylthioazetidin-2-one

The silyl protected acetoxy-azetidinone (23) (Preparation 4c, 4g) was dissolved in dry THF (70 ml) and 18- Crown-6 (98 mg) added. Powdered potassium fluoride (760 mg; 2 eq) was added and the mixture stirred at room temperature.

During the next five days more 18-Crown-6 (200 mg and 230 mg) was added as well as potassium fluoride (200 mg and 360 mg). Monitoring the reaction by t.e.c. showed slow disappearance of starting material.

The reaction mixture was diluted with brine and extracted with ethyl acetate. The ethyl acetate extracts were combined and washed with brine, dried ($MgSO_4$) and evaporated to give a gum.

Chromatography (silica gel/ethyl acetate-n-hexane) gave the title compound (24), 2.64g, m.p. 184-186°; νmax ($CHCl_3$) 3400,1775 $cm^{-1}$. ∂ ($CDCl_3$) 2.1 (3H, s); 3.85 (1H, dd, J=5, 2.5Hz); ca 4.2 (1H, br); 4.55 (1H, d, J=2.5Hz); 6.18 (1H, d, J=5Hz); 7.1-7.5 (ca 15H, m); 8.55 -8·7 (3H, m).

Preparation 4(e)

(3RS, 4SR) 3-[Acetoxy(pyrazinyl)methyl]-1-(1-p-nitrobenzyl-oxycarbonyl-1-triphenylphsphoranylidenemethyl)-4-tritylthio-azetidin-2-one

p-Nitrobenzyl glyoxylate monohydrate (1.16g) was suspended in dry benzene (80 ml) and boiled under reflux with a Dean Stark apparatus for removal of water (using 4A molecular sieve). After 1 hour the clear solution was cooled to room temperature and added to a solution of the azetidinone (24) (2.3g) from preparation 4d in THF (15 ml). Triethylamine (0.1 ml) was added and the solution allowed to stand at room temperature for 3 hours. Evaporation gave a crude hydroxy-ester as a foam, 3.6g; max ($CHCl_3$) 3300-3600, 1775, 1760 sh. $cm^{-1}$.

A solution of the hydroxy-ester (3.3g) in dichloromethane (50 ml) containing triethylamine (0.78 ml) and 4-dimethylaminopyridine (50 mg) was cooled to $0^o$. A solution of methanesulphonyl chloride (0.37 ml) in dichloromethane (10 ml) was then added dropwise with stirring. The solution was stirred at $0^o$ for 90 minutes and allowed to warm to room temperature for 30 minutes. Evaporation of solvent gave a dark coloured gum to which was added dry dioxan (5 ml), 2,6-lutidine (1.1 ml) and triphenylphosphine (5g, 4eq). The resulting mixture was heated at $40^o$ under argon for 17 hours.

The reaction mxiture was dissolved in ethyl acetate (100 ml) and washed with 1NHCl (50 ml x 3), aqueous sodium hydrogen carbonate and with brine. Drying ($MgSO_4$) and evaporation gave a dark, red gum which was chromatographed on silica eluting with ethyl acetate-n-hexane.

The title phosphorane (25) was obtained as a yellow foam, 1.75g; νmax ($CHCl_3$) 1750, 1600-1620 $cm^{-1}$.

Preparation 4(f)

(3RS, 4SR) Silver 3[Acetoxy(pyrazinyl)methyl]-1-(1-p-nitro-benzyloxycarbonyl-1-triphenylphosphoranylidenemethyl)azetidin-2-one-4-thiolate

The phosphorane (25) (1.87g) from preparation 4(e) was dissolved in dichloromethane (2 ml) - methanol (10 ml) and pyridine (0.2 ml) added. A solution of silver nitrate in methanol (0.15M; 15 mls) was added dropwise with stirring.

After 1 hour the yellow precipitate was removed by centrifugation and washed with ether. The thiolate (26) was obtained as a pale yellow solid, 1.46g; νmax ($CHCl_3$) 1745, 1600-1620 $cm^{-1}$.

Preparation 4(g)

(5RS, 6SR) p-Nitrobenzyl 6-[Acetoxy(pyrazinyl)methyl]penem-3-carboxylate

The silver thiolate (26) (0.9g) from preparation 4(f) was dissolved in dry dichloromethane (15 ml) and cooled in an ice bath. Triethylamine hydrochloride (750 mg; 5 eq) and 4-dimethylaminopyridine (150 mg; 1.1eq) were added followed by a solution of acetic formic anhydride (0.9 ml; 10 eq) in dichloromethane (4 ml).

The mixture was stirred at $0^{o}$ for 30 minutes and a precipitate formed. The precipitate was removed by filtration and washed well with ethyl acetate. The filtrate was washed with 1N hydrochloric acid, aqueous sodium hydrogen carbonate and with brine. Drying ($MgSO_4$) gave an ethyl acetate solution which was diluted to a volume of ca. 200 ml and heated at $40^{o}$ under argon for 1 hour.

Evaporation gave a gum which was chromatographed (silica gel eluted with ethyl acetate-n-hexane). The penem (27), 0.322g, had m.p. 148-149$^{o}$d when recrystallized from ethyl acetate-n-hexane; $\nu$ max ($CHCl_3$) 1790, 1740, 1720 $cm^{-1}$; $\delta$($CDCl_3$) 2.12 (3H, s); 4.51 (1H, m); 5.23 (1H, A, J=13Hz); 5.43 (1H, B, J=13Hz); 6.04 (1H, d, J=2Hz); 6.33 (1H, d, J=5Hz); 7.30 (1H, brs); 7.5 -7.6 and 8.15 - 8.25 (together 4H, m); 8.5 - 8.75 (3H, m).

Example 4(a)

(5RS) p-Nitrobenzyl 6-Pyrazinylmethylenepenem-3-carboxylate

The penem (27) (228 mg) from Preparation 4g was dissolved in dry dichloromethane (20 ml) and cooled to -40°. 1,8-Diazabicyclo[5.4.0]undec-7-ene (DBU) (91 mg) in dry dichloromethane (1.5 ml) was added dropwise with stirring.

After 10 minutes the solution was washed with 1N hydrochloric acid and dichloromethane (30 ml) added. The organic phase was then further washed with aqueos sodium hydrogen carbonate and with brine. Drying ($MgSO_4$) and evaporation of solvent followed by addition of a small volume of ether gave yellow crystals of the title compound (28), 150 mg, m.p. 166-169°d; λmax (EtOH) 309 (Em, 18,600) 250 nm (Em, 19,800); νmax ($CHCl_3$) 1780, 1715 $cm^{-1}$; ∂($d_6$ acetone) 5.38 (1H, d, J=13.8Hz); 5.50 (1H, d, J=13.8Hz); 6.75 (1H, d, J=1.1Hz); 7.39 (1H, brs); 7.70 (1H, s); 7.77 and 7.80 (together 2H, two brs); 8.26 and 8.29 (together 2H, two brs); 8.66 (1H, d, J=2.4Hz); 8.75-8.77 (1H, m); 8.92 (1H, d, J=1.4Hz).

Example 4(b)



5(RS) Sodium 6-Pyrazinylmethylenepenem-3-carboxylate

The penem ester (28) (100 mg) was dissolved in $H_2O$ (8ml)-dioxan (32 ml) and added to a prehydrogenated suspension of 5% Pd/C (100 mg) in the same solvent (2 ml). The mixture was hydrogenated at atmospheric pressure and room temperature for 40 minutes.

Sodium hydrogen carbonate (17 mg) dissolved in a small volume of water was added to the reaction mixture and the catalyst removed by filtration. The catalyst was washed with $H_2O$-dioxan and the filtrate evaporated to a gum.

The crude product was purified by passage through a column of Biogel P2 eluted with water. Fractions containing the product were combined and freeze dried. The title compound (29) was obtained as a yellow-brown solid, 19 mg, λmax ($H_2O$) 299 (Em 12,200) 246 nm (Em 13.300); νmax (KBr) 1760, 1598 $cm^{-1}$; δ($D_2O$) 6.67 (1H, s); 7.04 (1H, s); 7.24 (1H, s); 8.51 (1H, d, J=2.4Hz); 8.69 (1H, brs); 8.73 (1H, brs).

Preparation 5

Methyl Pyrimidine-4-carboxylate

Pyrimidine-4-carboxylic acid (4.5 g) (Ber. 1960, 93, 2407) was suspended in methanol (100 ml) and conc. sulphuric acid (0.1 ml) added. The mixture was boiled under reflux for 48 hours, cooled and neutralized with sodium bicarbonate. Evaporation under reduced pressure gave a crude product which was diluted with brine and extracted with ethyl acetate. The extracts were dried ($MgSO_4$) and evaporated to dryness. Chromatography of the crude product on silica gel eluting with ethyl acetate-n-hexane gave the methyl ester, 2.5 g, m.p. 67 - 69°, $\nu_{max}$ ($CHCl_3$) 1735 $cm^{-1}$.

Preparation 5(a)

(3RS,4SR) N-t-Butyldimethylsilyl-3-(4-pyrimidinyl-carbonyl) -4-tritylthio-azetidin-2-one

Azetidinone (1) (6.9 g) was condensed with methyl pyrimidine-4-carboxylate (2.3 g) in the manner described in Preparation 4(a).

Chromatography of the crude product (silica gel eluted with ethyl acetate/n-hexane) gave the title compound (30), 6.3 g; m.p. 160 - 162°C decomp.; $\nu_{max}$ ($CHCl_3$) 1750, 1700 $cm^{-1}$; nmr $\delta$ ($CDCl_3$) 0.95 (9H, s); 4.93 (1H, d, J = 2 Hz); 5.05 (1H, d, J = 2 Hz); 6.9 - 7.5 (15H, m); 7.65 (1H, dd, J = 5, 1 Hz); 8.87 (1H, d, J = 5 Hz); 9.30 (H, d, J = 1 Hz); signal for $Me_2Si$ not observed. (Found: C, 69.84; H, 6.42; N, 7.39; S, 5.44; $C_{33}H_{35}N_3SSiO_2$ requires C, 70.05; H, 6.24; N, 7.43; S, 5.67%).

## Preparation 5(b)

## (3RS,4SR) N-t-Butyldimethylsilyl-3-[hydroxy(4-pyrimidinyl)-methyl]-4-tritylthio-azetidin-2-one

The azetidinone-ketone (30) (6.2 g) from Preparation 5(a) was treated with sodium borohydride (0.5 g) using the method described in Preparation 4(b).

Chromatography of the crude product gave two isomers:-
Isomer I (31), 1.35 g, crystallized from ethyl acetate/n-hexane had m.p. 188 - 190°; i.r. $\nu_{max}$ ($CHCl_3$) 3400 br, 1745 $cm^{-1}$; nmr $\delta$ ($CDCl_3$) 0.9 (9H, s); 3.35 (1H, brdd, J = 2.7, 1.8 Hz after $D_2O$ addition); 3.6 - 3.7 (1H, br, with $D_2O$ gives d, J = 2.7 Hz); 4.1 (1H, br, exch. $D_2O$); 4.62 (1H, brs, collapse to d, J = 1.8 Hz with $D_2O$); 6.94 (1H, d, J = 5 Hz); 7.2 (15H, m); 8.56 (1H, d, J=5Hz); 9.03 (1H, s).
Isomer II (32), 4.16 g, i.r. $\nu_{max}$ ($CHCl_3$) 3200 - 3500, 1730 $cm^{-1}$; nmr $\delta$ ($CDCl_3$) 0.8 (9H, s); 3.0 - 3.5 (1H, br, exch. $D_2O$); 3.71 (1H, dd, J = 6.2, 1.7 Hz); 4.32 (1H, br d, J = 6.2 Hz, sharpens with $D_2O$); 4.35 (1H, d, J = 1.7Hz); 7.1 - 7.5 (16H, m); 8.49 (1H, d, J = 5 Hz); 9.0 (1H, d, J ca. 1 Hz).

## Preparation 5(c)

## (3RS,4SR) 3-[Hydroxy-(4-pyrimidinyl)methyl]-4-tritylthio-azetidin-2-one

The N-silyl protected azetidinone (32) (isomer II; 4.1 g) was dissolved in methanol (70 ml) and cooled to -30°. A solution of potassium fluoride (0.55 g) in methanol (15 ml) was added dropwise with stirring. Over a period of 1 hour the solution was allowed to warm to 0° and tlc showed disappearance of starting material.

The solution was diluted with brine (100 ml) and extracted with ethyl acetate (100 ml x 2). The extracts were washed with brine, dried ($MgSO_4$) and evaporated to give a foam which was dried further by azeotroping with chloroform.

Chromatography on silica (EtOAC/hexane) gave the title compound (33) as a white solid, 3.4 g (as a chloroform solvate), i.r. $\nu_{max}$ ($CHCl_3$) 3100 - 3600, 3400 sh, 1760 $cm^{-1}$; nmr $\delta$ ($d_6$-acetone) 3.83 (1H, dd, J = 2, 2 Hz); 4.40 (1H, d, J = 2 Hz); 5.09 (1H, dd, J = 7,2 Hz); 5.25 (1H, d, J = 7 Hz); 5.45 (1H, br s); 7.25 (15H, br); 7.66 (1H, d, J = 6 Hz); 8.93 (1H, d, J = 6 Hz); 9.15 (1H, s); also signal for $CHCl_3$.

A sample of (33) crystallized from ethyl acetate/n-hexane had m.p. 182 - 186°. (Found: C, 71.48; H, 5.02; N, 9.07; S, 6.90; $C_{27}H_{23}N_3O_2S$ requires C 71.50; H, 5.11; N, 9.26; S, 7.07%).

<u>Preparation 5(d)</u>

<u>(3RS,4SR) 3-[Acetoxy-(4-pyrimidinyl)methyl]-4-tritylthio azetidin-2-one</u>

The azetidinone (33) (3.35 g of chloroform solvate) from Preparation 5(c) was dissolved in dry methylene chloride (35 ml) and triethylamine (0.93 ml) was added followed by 4-dimethylaminopyridine (50 mg). The solution was cooled in an ice bath and acetic anhydride (0.59 ml) added. After 40 minutes at 0° the solution was washed with 1N HCl, aq. $NaHCO_3$ and with brine. Drying ($MgSO_4$) and evaporation gave a foam. Chromatography gave the title acetate (34), 2.8 g, m.p. 185 - 187°; i.r. $\nu_{max}$ ($CHCl_3$) 3380, 1770 $cm^{-1}$; nmr $\delta$ ($CDCl_3$) 2.12 (3H, s); 3.83 (1H, dd,

J = 5, 3 Hz); 4.25 (1H, br s); 4.49 (1H, d, J = 3 Hz); 6.04 (1H, d, J = 5 Hz); 7.15 - 7.45 (16H, m); 8.78 (1H, d, J = 5 Hz); 9.27 (1H, br s). (Found: C, 69.95; H, 5.12; N, 8.12; S, 6.44; $C_{29}H_{25}N_3O_3S$ requires C, 70.28; H, 5.08; N, 8.48,; S, 6.47%).

Preparation 5(e)

(3RS,4SR) 3-[Acetoxy-(4-pyrimidinyl)methyl]-1-(1-p-nitrobenzyloxycarbonyl-1-triphenylphosphoranylidenemethyl)-4-tritylthio-azetidin-2-one

p-Nitrobenzyl glyoxylate monohydrate (1.31 g) was suspended in dry benzene and boiled under reflux with a Dean Stark apparatus for removal of water. After 1 hour the clear solution was cooled to room temperature and a solution of azetidinone (34) (2.6 g) in dry THF (15 ml) was added. Triethylamine (0.05 ml) was added to the solution which was left at room temperature for 3 hours and disappearance of starting material was monitored by tlc. Evaporation of solvent gave a crude hydroxy-ester as a foam, 4.1 g.

The above hydroxy-ester was dissolved in dry THF (60 ml) and 2,6-lutidine (0.76 mls) was added. The solution was cooled to -25° and a solution of thionyl chloride (0.45 ml) in THF (10 ml) was added dropwise with stirring.

After 30 minutes the precipitate was removed by filtration and the filtrate evaporated to a dark-coloured foam. This crude chloro-ester was essentially one spot on tlc.

Triphenylphosphine (5.5g)was added to the chloro-ester and the mixture dissolved in dry dioxan (12 ml). The mixture was concentrated by evaporation under reduced pressure and 2,6-lutidine (1.5 ml) added.

The mixture was heated at 40° under argon for 24 hours and then left at room temperature for ca. 17 h. The mixture was diluted with ethyl acetate and washed with 1N HCl, aqueous. $NaHCO_3$ and with brine. Drying ($MgSO_4$) and evaporation gave a gum which was purified by column chromatography.

The title phosphorane (35) was obtained as a buff coloured foam, 3.2 g, i.r. $\nu_{max}$ ($CHCl_3$) 1750, 1600-1620 $cm^{-1}$.

## Preparation 5(f)

### (3RS,4SR) Silver 3-[acetoxy-(4-pyrimidinyl)methyl]-1-(p-nitrobenzyloxycarbonyl-1-triphenylphosphoranylidenemethyl) azetidin-2-one-4-thiolate

The phosphorane (35) (3.54 g) from Preparation 5(e) was treated with silver nitrate in methanol as described in Preparation 4(f).

Filtration of the precipitated solid and ether washing gave the desired silver salt (36), 2.42 g; ir $\nu_{max}$ ($CHCl_3$) 1745, 1600 - 1620 $cm^{-1}$.

## Preparation 5(g)

### (5RS,6SR) p-Nitrobenzyl 6-[acetoxy-(4-pyrimidinyl)methyl]-penem-3-carboxylate

Using the method described in Preparation 4(g), silver thiolate (36) (1.55 g) from Preparation 5(f) was converted to the desired title compound (37) which was purified by repeated chromatography on silica gel (EtOAc/n-hexane and EtOAc/$CH_2Cl_2$).

The title penem (37) was obtained as an off-white solid, 505 mg, uv $\lambda_{max}$ (EtOH) 254 (E 14,600), 313nm (E 8,400); i.r. $\nu_{max}$ ($CHCl_3$) 1795, 1740 sh, 1720 $cm^{-1}$; nmr $\delta$ ($CDCl_3$) 2.18 (3H, s); 4.54 (1H, ddd, J = 4, 2.2, 1 Hz); 5.24 (1H, A, J = 13 Hz); 5.42 (1H, B J = 13 Hz); 6.20 (1H, d, J = 4 Hz); 7.28) (1H, d, J = 1.1 Hz); 7.38 (1H, dd, J = 6, 1 Hz); 7.45 - 7.6 (2H, aromatic); 8.15 - 8.25 (2H, aromatic); 8.75 (1H, d, J = 6 Hz); 9.16 (1H, d, J = 1 Hz).

Example 5(a)

(5RS) p-Nitrobenzyl 6-(4-pyrimidinylmethylene)penem-3-carboxylate

The penem (37) (480 mg) from Preparation 5(g) was treated with DBU using the method of Example 4a. The crude product was purified by silica gel chromatography eluting with ethyl acetate/methylene dichloride. The title compound (38) was obtained as a yellow crystalline solid, 294 mg, m.p. >160° decomp. (ex. $CHCl_3$/ether); uv $\lambda_{max}$ (EtOH) 261 (E 22,308), 283 nm (E 22,000); ir $\nu_{max}$ ($CHCl_3$) 1775, 170 br $cm^{-1}$; nmr $\delta$ ($d_6$ acetone) 5.38 (1H, d, J = 14 Hz); 5.50 (1H, d, J = 14 Hz); 6.80 (1H, d, J = 1 Hz); 7.28 (1H, br s); 7.73 (1H, s); 6.90 (1H, d, J = 1 Hz); 8.26 - 8.29 (2H,. m); 8.94 (1H, d, J = 5 Hz); 9.25 (1H, J = 1.2 Hz). (Found; C, 54.61; H, 3.17; N, 13.99; S 7.94 $C_{18}H_{12}N_4O_5S$ requires C, 54.54; H, 3.05; N, 14.14; S, 8.09%).

Example 5(b)

(5RS) Sodium 6-(4-pyrimidinylmethylene)penem-3-carboxylate

Penem (38) (29 mg) from Example 5(a) was dissolved in dioxan (15 ml) - water (3 ml). 1% Aqueous sodium bicarbonate (0.62 ml) was added to the solution followed by 5% Pd/C (36 mg) and the mixture hydrogenated at atmospheric pressure/room temperature for 15 minutes.

Catalyst was removed by filtration and the filtrate evaporated to dryness. Crude product was purified by passage through a column of Biogel P2 eluted with water. Column fractions were combined on the basis of their uv spectra and freeze-dried to give the title compound (39), 7.5 mg, $\lambda_{max}$ ($H_2O$) 285 (E 11,900), 248.5 nm (E 10,300); $\nu_{max}$ (KBr) 1760, ca. 1590 br $cm^{-1}$; nmr $\delta$ ($D_2O$) 6.72 (1H, d, J ca. 1 Hz); 7.06 (1H, s); 7.15 (1H, br s); 7.61 (1H, dd, J = 5, ca 1 Hz); 8.78 (1H, d, J = 5 Hz); 9.16 (1H, br d, J ca. 1 Hz), irradiation at 7.15 causes signal at 6.72 to collapse to a singlet.

Preparation 6(a)

(3RS, 4SR) 1-t-Butyldimethylsilyl-3-[hydroxy-(4-pyridyl)-methyl]-4-tritylthioazetidin-2-one

4-Pyridinecarboxaldehyde (2.4 g) was condensed with 1-t-butyldimethylsilyl-4-tritylthioazetidin-2-one (9.2g) in the manner described in the Preparation 1(a).

Chromatography (silica gel eluted with ethyl acetate/ n-hexane) of the crude product gave the title compound (40), 9.4 g; m.p. 170-171°C; ir $\nu_{max}$ ($CHCl_3$) 1730 $cm^{-1}$; nmr $\delta$($CDCl_3$ + $D_2O$) 0.75 (9H, s); 3.73 (1H, d.d., J=6.2, 1.8Hz); 3.93 (1H, d, J=1.8Hz); 4.10 (1H, d, J=6.2Hz); 6.83 (2H, m);

7 .30 (15H, m); 8.35 (2H, m). (Found: C, 71.82; H, 6.63; N, 4.83; S, 5.47; $C_{34}H_{38}N_2O_2SSi$ requires C, 72.08; H, 6.71; N, 4.95; S, 5.65%).

Preparation 6(b)

(3RS, 4SR) 3-[Hydroxy-(4-pyridyl)methyl]-4-tritylthio-azetidin-2-one

A solution of anhydrous potassium fluoride (1.25 g) in methanol (100 ml) was added to a stirred solution of the silyl protected hydroxy-azetidinone (40) (Preparation 6(a), 9.35 g) in methanol (300 ml) at -10°C. After stirring at -10°C for 0.5 hour followed by a further 0.5 hour to 0°C the title compound (41) crystallised from solution and was filtered off, dried. Yield 5.44 g, m.p. 131°C; i.r. $\nu_{max}$ ($CHCl_3$) 3400, 1760 $cm^{-1}$; nmr $\delta$($CDCl_3$) 3.48 (1H, t, J=2.9Hz); 4.30 (1H, s); 4.41 (1H, d, J=2.9Hz); 4.10-4.55 (1H, br); 5.14 (1H, d, J=2.9Hz); 7.05-7.50 (17H, m); 8.63 (2H, m). (Found: C, 74.01; H, 5.36; N, 6.29; S, 6.83; $C_{28}H_{24}N_2O_2S$ requires C, 74.34; H, 5.31; N, 6.19; S, 7.07%).

Preparation 6(c)

(3RS, 4SR) 3-[Acetoxy-(4-pyridyl)methyl]-4-tritylthio-azetidin-2-one

The deprotected azetidinone (41) (Preparation 6(b), 6.38g) was dissolved in dry dichloromethane (200 ml) containing 4-dimethyl aminopyridine (1.90g; 1.1 equiv.). The solution was cooled in an ice bath and acetic anhydride (1.56 ml; 1.2 eq.) was added. The reaction mixture was stirred for 0.5 hour when t.l.c. indicated no starting material remained. The solution was washed with dilute

hydrochloric acid, with aqueous sodium hydrogen carbonate and with brine. Drying ($MgSO_4$) and evaporation gave a crude product which was chromatographed (silica gel-ethyl acetate).

The acetate (42), 6.8 g, had a melting point, 190-193°C when crystallised from dichloromethane; $\nu_{max}$ ($CHCl_3$) 3400, 1770 $cm^{-1}$; n.m.r. $\delta$($CDCl_3$) 2.10 (3H, s); 3.56 (1H, dq, J=1.0, 2.9, 5.6Hz); 4.30 (1H, d, J=2.9Hz); 4.37 (1H, s); 6.03 (1H, d, J=5.6Hz); 7.05-7.50 (17H, m); 8.55-8.75 (2H, m); (Found: C, 72.61; H, 5.13; n, 5.65; S, 6.30; $C_{30}H_{26}N_2O_3S$ requires C, 72.87; H, 5.26; N, 5.67; S, 6.48%).

Preparation 6(d)

(3RS, 4SR) 3[Acetoxy-(4-pyridyl)methyl]-1-(1-p-nitrobenzyloxy-carbonyl-1-triphenylphosphoranylidene-methyl)-4-trityl-thioazetidin-2-one

p-Nitrobenzyl glyoxylate (1.58 g) was suspended in dry benzene (80 ml) and boiled under reflux with a Dean Stark apparatus for removal of water. After 1 hour the clear solution was cooled to room temperature and added to a solution of the azetidinone (42) (3.13 g) from Preparation 6(c) in THF (30 ml). Triethylamine (0.06 ml) was added and the solution allowed to stand at room temperature for 2 hours. Evaporation gave a crude hydroxyester as a foam, 4.70g; $\nu_{max}$ ($CHCl_3$) 1760 $cm^{-1}$.

A solution of the hydroxy ester (8.63 g) in dry THF (100 ml) was cooled to 0°C, and 2,6-lutidine (1.72 ml) added. A solution of thionyl chloride (1.0 ml) in dry THF (20 ml) was then added dropwise with stirring and the reaction continued for 1.0 hour at 0°C. Evaporation of the

solvent gave a coloured foam to which was added dry dioxan (8 ml), 2,6-lutidine (2.14 ml) and triphenylphosphine (9.66g, 3 eq.). The resulting mixture was heated at 60°C under an atmosphere of argon for 5 hours.

The reaction mixture was dissolved in ethyl acetate (300 ml) and washed with 1N HCl (100 ml), aqueous sodium bicarbonate and with brine. Drying with $MgSO_4$ and evaporation gave a dark red foam which was chromatographed on silica eluting with ethyl acetate/n-hexane.

The title phosphorane (43) was obtained as a yellow foam, 5.65g; $\nu_{max}$ ($CHCl_3$) 1745, 1600-1620 $cm^{-1}$.

Preparation 6(e)

(3RS; 4SR) Silver 3[acetoxy-(4-pyridyl)methyl]-1-(1-p-nitrobenzyloxycarbonyl-1-triphenylphosphoranylidene-methyl)azetidin-2-one-4-thiolate

The phosphorane (43) (5.65 g) from Preparation 6(d) was dissolved in dichloromethane (15 ml) methanol (45 ml) and pyridine (0.63 ml) added. A solution of silver nitrate in methanol (0.15M; 48 ml) was added dropwise with stirring.

After 1.0 hour at ambient followed by 0.5 hour at 0°C the resulting yellow precipitate was separated by filtration and washed with ether. The thiolate (44) was obtained as a pale yellow solid, 4.10 g; $\nu_{max}$ ($CHCl_3$) 1750, 1600-1620 $cm^{-1}$.

Preparation 6(f)

(5RS, 6SR) p-Nitrobenzyl 6-[acetoxy-(4-pyridyl)methyl]penem -3-carboxylate

The silver thiolate (44) (2.17 g) from Preparation 6(e) was dissolved in dry dichloromethane (35 ml) and cooled in an ice bath. 4-Dimethylaminopyridine (0.358 g; 1.1 eq.) and acetic formic anhydride (2.13 ml; 10 eq) were added followed by triethylaminehydrochloride (1.84g; 5eq). The mixture was stirred 5 minutes at $0^oC$ when a precipitate formed. The ice bath was removed and stirring continued for 20 minutes. The precipitate was removed by filtration and washed well with ethyl acetate. The filtrate was washed with 1N HCl, aqueous sodium bicarbonate and with brine. Drying ($MgSO_4$) gave an ethyl acetate solution which was diluted to a volume of 500 ml and heated at $40^oC$ under argon for 1 hour.

Evaporation gave a gum which was chromatographed (silica gel eluted with ethyl acetate-n-hexane) to give the title penem (45) (0.75g) as a $3^+:1^o$ isomeric mixture contaminated with triphenylphosphine oxide $\lambda_{max}$ (EtOH) 259 ($\varepsilon$m 15000) and 314 nm ($\varepsilon$m 7700); $\nu_{max}$ ($CHCl_3$) 1755 (sh), 1740 $cm^{-1}$; $\delta$ppm ($CDCl_3$) $2.19^+$ (s) and $2.20^o$ (s) [3H]; 4.24 to 4.29 (1H, m); 5.24 to 5.43 (2H, q, J=14,33Hz); $5.62^o$ (¼H, d, J=2Hz); $5.75^+$ (3/4H, d, J=2Hz); 6.16 (1H, m); 7.23 to 7.30 (2½H, m); $7.53^o$ (½H, m); 7.57 (2H, m); 8.23 (2H, m); 8.66 (2H, m).

Example 6(a)

(5RS) p-Nitrobenzyl 6-[4-pyridylmethylene]penem-3-carboxylate

The penem (45) (710 mg) from Preparation 6(f) was treated with DBU (302 mg, 1.2 eq) as for Example 1(a) to

give the title penem (46) (378 mg) as a mixture of (Z) and (E) isomers. Crystallisation of this isomeric mixture from methylene dichloride/ethyl acetate/n-hexane gave a single (Z)-isomer having a m.p. 171-172°C; $\lambda_{max}$ (EtOH) 266 nm (εm 31311); $\nu_{max}$ ($CHCl_3$) 1785, 1720 $cm^{-1}$; δppm ($CDCl_3$) 5.40 (2H, m); 6.65 (1H, s); 7.12 (1H, s); 7.17 (2H, m); 7.37 (1H, s); 7.62 (2H, m); 8.24 (2H, m); 8.73 (2H, m). (Found C, 57.85; H, 3.18; N, 10.66 ; $C_{19}H_{13}N_3O_5S$ requires C, 57.72; H, 3.29; N, 10.63%).

Example 6(b)

(5RS) Sodium 6-(4-pyridylmethylene)penem-3-carboxylate

The penem ester (46) (a mixture of Z and E isomers) from Example 6(a) (113 mg) was dissolved in a mixture of dioxan (24 ml) and water (6 ml) and was hydrogenated over 5% palladium/charcoal catalyst (150 mg) at S.T.P. for 30 minutes. A 1% sodium bicarbonate solution (2.5 ml) was added and the mixture filtered through a celite pad. The pad was washed thoroughly with water, and the filtrate concentrated to a small volume (5 ml). The concentrated filtrate chromatographed on Biogel P2 eluting with water. The appropriate fractions were freeze-dried to give the title penem (47) (34 mg) as a 7:1 mixture of (Z):(E) isomers. $\lambda_{max}$ ($H_2O$) 267 nm (εm 17700); $\nu_{max}$ (KBr) 1765, 1600 $cm^{-1}$; δppm ($D_2O$) 6.48 (1/8H, s); 6.80 (7/8H, s); 6.88 (1/8H, s); 7.09 (7/8H, s); 7.18 (1/8H, s); 7.21 (7/8H, s); 7.42 (2H, m); 8.59 (2H, m).

Preparation 7(a)

(3RS, 4SR) N-t-Butyldimethylsilyl-3-(6-methoxy-3-pyridyl)carbonyl-4-tritylthio-azetidin-2-one

1-t-butyldimethylsilyl-4-tritylthioazetidin-2-one (1) (9.2 g) was condensed with 3-carbethoxy-6-methoxy-pyridine

(4.09 g) (N.M. Chung and H. Tieckelmann, J.Org.Chem. 35, No. 8, p. 2517) as described in Preparation 4(a).

Chromatography (silica gel eluted with ethyl acetate/n-hexane) gave the title azetidinone (48) (9.22 g) $\nu_{max}$ ($CHCl_3$) 1740, 1665, 1590 $cm^{-1}$; $\delta$ppm ($CDCl_3$) 0.39 (6H, s); 0.99 (9H, s); 4.00 (4H, m); 5.14 (1H, m); 6.60-8.03 (18H, m); 8.50 (1H, m).

## Preparation 7(b)

### (3RS, 4SR) N-t-Butyldimethylsilyl-3-[hydroxy(6-methoxy-3-pyridyl)methyl]-4-tritylthio-azetidin-2-one

The azetidinone-ketone (48) from Preparation 7(a) (9.22 g) was dissolved in dioxan (100 ml) containing pH 7 phosphate buffer (10 ml). Sodium borohydride (1.53 g) was added in portions with stirring over 2.0 hours and allowed to continue stirring 2.5 hours.

The solution was diluted with brine and with ethyl acetate (*ca* 250 ml). The organic phase was separated, washed with brine, dried ($MgSO_4$) and evaporated. Chromatography (silica gel eluted with ethyl acetate/n-hexane) gave the title azetidinone (49) (7.0 g) which when crystallised from ethyl acetate/n-hexane had m.p. 149°C. $\nu_{max}$ ($CHCl_3$) 1740 (sh), 1725, 1605 $cm^{-1}$; $\delta$ppm ($CDCl_3$) -0.05 (3H, s); 0.00 (3H, s); 0.77 (9H, s); 2.34 (1H, d, J=8.0Hz); 3.73 (1H, dd, J=6.4, 1.8Hz); 3.90 (5H, m); 6.61 (1H, d, J=8.5Hz); 7.20-7.50 (16H, m); 7.61 (1H, d, J=2.3Hz). (Found:C 70.34; H, 6.57; N, 4.55; S, 5.13. $C_{35}H_{40}N_2O_3SSi$ requires: C, 70.47; H, 6.71; N, 4.70; S, 5.37%).

Preparation 7(c)

(3RS, 4SR) 3-[Hydroxy(6-methoxy-3-pyridyl)methyl]-4-tritylthioazetidin-2-one

The silyl-protected hydroxy-azetidinone (49) (Preparation 7(b), 7.0 g) was deprotected as described in Preparation 5(c).

Chromatography (silica gel eluted with ethyl acetate-n-hexane) gave the title azetidinone (50), 5.56 g, m.p. 198°C; $\nu_{max}$ ($CHCl_3$) 3,400, 1755 $cm^{-1}$; δppm ($CDCl_3$ + $D_2O$) 3.45 (1H, m); 3.97 (3H, s); 4.30 (1H, s); 4.50 (1H, d, J=3Hz); 5.13 (1H, d, J=5Hz); 6.80 (1H, d, J=11Hz); 7.25 (15H, m); 7.57 (1H, dd, J=11Hz, 2Hz); 8.22 (1H, d, J=2Hz).

Preparation 7(d)

(3RS, 4SR) 3-[Acetoxy(6-methoxy-3-pyridyl)methyl]-4-tritylthioazetidin-2-one

The deprotected azetidinone (50) (Preparation 7(c), 5.56 g) was acetylated as described in Preparation 5(d). Chromatography (silica gel eluted with ethyl acetate/n-hexane) gave the title azetidinone (51) 5.31 g as a white foam, $\nu_{max}$ ($CHCl_3$) 3400, 1765 $cm^{-1}$; δppm ($CDCl_3$) 2.12 (3H, s); 3.60 (1H, m); 3.97 (3H, s); 4.33 (1H, s); 4.40 (1H, d, J=3.0Hz); 6.04 (1H, d, J=6.0Hz); 6.75 (1H, d, J=9.0Hz); 7.20 to 7.60 (16H, m); 8.11 (1H, d, J=2.0Hz).

A sample of (51) crystallised from ethyl acetate/n-hexane had a m.p. 158-159°C (Found C, 70.89; H, 5.43; N, 5.41; S, 6.07; $C_{37}H_{28}N_2O_4S$ requires C, 70.99; H, 5.34; N, 5.34; S, 6.10%).

Preparation 7(e)

(3RS, 4SR) 3-[Acetoxy(6-methoxy-3-pyridyl)methyl]-1-(1-p-nitrobenzyloxycarbonyl-1-triphenylphosphoranylidenemethyl)-4-tritylthio-azetidin-2-one

The azetidinone (51) (5.1 g) from Preparation 7(d) was reacted with p-nitrobenzylglyoxylate (2.55 g, 1.1 eq) as described in Preparation 6(d) to give the crude hydroxy ester as a foam, 7.60 g, $\nu_{max}$ ($CHCl_3$) 1760 $cm^{-1}$.

A solution of the hydroxy ester (7.60 g) in dry THF (80ml) was cooled to -10°C and 2,6-lutidine (1.41 ml) added. A solution of thionyl chloride (0.83 ml) in dry THF (10 ml) was added dropwise with stirring and the reaction continued 0.5 hour at -10°C. Filtration and evaporation of the solvent gave a foam to which was added dry dioxan (8 ml), triphenylphosphine (7.90 g, 3 eq), and 2,6-lutidine (1.76 ml). The resulting mixture was stirred at 40°C under an atmosphere of argon for 60 hours.

The reaction mixture was dissolved in ethyl acetate (300 ml) and washed with 1N HCl (100 ml), aqueous sodium bicarbonate solution and with brine. Drying ($MgSO_4$) and evaporation of the solvent gave a coloured foam which was chromatographed on silica gel eluting with ethyl acetate/n-hexane. The title phosphorane (52) was obtained as a foam, 6.8g $\nu_{max}$ ($CHCl_3$) 1750, 1610 $cm^{-1}$.

Preparation 7(f)

(3RS, 4SR) Silver 3-[acetoxy(6-methoxy-3-pyridyl)methyl]-1-(1-p-nitrobenzyloxycarbonyl-1-triphenyl-phosphoranylidene-methyl)azetidin-2-one-4-thiolate.

The phosphorane (52) (6.8 g) from the Preparation 7(e) was reacted with methanolic silver nitrate in the manner described in Preparation 6(e).

The thiolate (53) was obtained as a yellow solid, 5.70 g, $\nu_{max}$ ($CHCl_3$) 1740, 1610 $cm^{-1}$.

Preparation 7(g)

(5RS, 6SR) p-Nitrobenzyl 6-[acetoxy(6-methoxy-3-pyridyl) methyl]penem-3-carboxylate

The thiolate (53) (2.5g) from the Preparation 7(f) was cyclised in the manner described in the Prepartion 6(f).

Chromatography (silica gel eluted with ethyl acetate-n-hexane) gave the title penem (54), 1.2 g. $\nu_{max}$ ($CHCl_3$) 1800, 1740 (sh), 1720, 1610 $cm^{-1}$; $\delta$ppm ($CDCl_3$) 2.12 (3H, s); 3.90 (3H, s); 4.30 (1H, dd, J=2Hz, 6Hz); 5.26 (1H, A, J=13Hz); 5.33 (1H, B, J=13Hz); 5.77 (1H, d, J=2Hz); 6.14 (1H, d, J=6Hz); 6.78 (1H, d, J=9Hz); 7.30 (1H, s); 7.50-7.70 (3H, m); 8.15 (1H, d, J=2.5Hz); 8.25 (2H, m).

Example 7(a)

(5RS) p-Nitrobenzyl 6(6-methoxy-3-pyridyl)methylene-penem -3-carboxylate.

The penem (54) (536 mg) from Preparation 7(g) was reacted with DBU in the manner described in Example 1(a).

Chromatography (silica gel eluting with ethyl acetate/ n-hexane) gave two isomeric penems, the (E)-isomer of the title compound (55) 52 mg and the (Z)-isomer (56) 274 mg both as yellow solids. The (E)-isomer (55) gave $\lambda_{max}$(EtOH) 288 ($\varepsilon$m 20037), 316nm ($\varepsilon$m 26694); $\nu_{max}$ ($CHCl_3$) 1780, 1715 $cm^{-1}$; $\delta$ppm ($CDCl_3$) 4.00 (3H, s); 5.40 (2H, m); 6.44 (1H, s); 6.60 (1H, s); 6.84 (1H, d, J=8.8Hz); 7.45 (1H, s); 7.63 (2H, d, aromatic); 8.26 (2H, d, aromatic); 8.34 (1H, d, J=2.4Hz); 8.65 (1H, dd, J=8.8, 2.4Hz).

The (Z)-isomer (56) gave $\lambda_{max}$ (EtOH) 290 ($\varepsilon$m 17230) 311 ($\varepsilon$m 20530); $\nu_{max}$ ($CHCl_3$) 1780; 1715 $cm^{-1}$; $\delta$ppm ($CDCl_3$) 3.99 (3H, s); 5.37 (2H, m); 6.61 (1H, s); 6.83 (1H, d, J=8.7Hz); 7.13 (1H, s); 7.34 (1H,s), 7.48 (1H, dd, J=8.7 and 2.0Hz); 7.62 (2H, d, aromatic); 8.25 (3H, m, including 2 x aromatic).

Example 7(b)

(5RS) (6E) Sodium 6-(6-methoxy-3-pyridyl)methylene-penem -3-carboxylate

The penem ester (55) (47 mg) from Example 7(a) was hydrogenolysed and converted to the sodium salt as described in Example (6b).

The title compound (57) was isolated as a yellow solid, 7 mg, $\lambda_{max}$ ($H_2O$) 291 nm ($\varepsilon$m 10700) 309 nm ($\varepsilon$m 11700); $\delta$ppm ($D_2O$) 3.93 (3H, s); 6.44 (1H, s); 6.79 (1H, s); 6.92 (1H, d, J=9.0Hz); 7.13 (1H, s); 8.38 (1H, d, J=2.3Hz); 8.44. 8.47 (1H, dd, J=9.0Hz and 2.3 Hz).

Example 7(c)

(5RS) (6Z) Sodium 6-(6-methoxy-3-pyridyl)methylene-penem-3-carboxylate

The penem ester (56) (176 mgs) from Example 7(a) was hydrogenolysed and converted to the sodium salt as described in Example 6(b).

The title compound (58) was isolated as a yellow solid, 80 mg, $\lambda_{max}$ ($H_2O$) 293nm ($\varepsilon$m 19990), 307nm ($\varepsilon$m 21200); $\nu_{max}$ (KBr) 1760, 1675, 1600 $cm^{-1}$; $\delta$ppm ($D_2O$) 3.92 (3H, s); 6.65 (1H, bs); 6.90 (1H, bd, J=8.5Hz); 7.01 (1H, s); 7.06 (1H, bs); 7.64 (1H, bd, J=3Hz); 8.11 (1H, bs).

Preparation 8(a)

(3RS, 4SR) 1-t-Butyldimethylsilyl-3-[hydroxy-(3-pyridazinyl)methyl]-4-tritylthioazetidin-2-one

3-Pyridazine carboxaldehyde (0.22 g) [G. Heinisch and A. Mayrhojer, Monatshefte fuer Chemie, 108, 213-224 (1977)] was condensed with 1-t-butyldimethylsilyl-4-tritylthio azetidin-2-one (1) (0.92 g) as described in Preparation 1(a).

Chromatography (silica gel eluted with ethyl acetate-n-hexane) of the crude product gave the title azetidinone (59) 0.60 g as a foam, $\nu_{max}$ ($CHCl_3$) 3400 (b), 1745 $cm^{-1}$; $\delta$ppm ($CDCl_3$) 0.79 (9H, s), 3.69 (1H, dd, J=6Hz, 2Hz); 4.35 (1H, d, J=2Hz); 4.52 (1H, d, J=6Hz); 7.32 (17H, m); 8.98 (1H, dd, J=5Hz, 2Hz).

Preparation 8(b)

(3RS, 4SR) 3-[Hydroxy-(3-pyridazinyl)methyl]-4-tritylthio-azetidin-2-one

The silyl azetidinone (59) (Preparation 8(a), 1.14 g) was deprotected in the manner described in Preparation 6(b).

Chromatography (silica gel eluted with ethyl acetate) gave the title azetidinone (60) 0.85 g. $\nu_{max}$ ($CHCl_3$) 3400, 3350 (b), 1765 $cm^{-1}$; $\delta$ppm ($CDCl_3$) 3.65 (1H, m); 4.22 (1H, bs); 4.28 (1H, s); 4.51 (1H, d, J=3Hz); 5.32 (1H, d, J=5Hz); 7.00-7.80 (17H, m); 9.21 (1H, dd, J=5Hz, 2Hz).

Preparation 8(c)

(3RS, 4SR) 3[Acetoxy-(3-pyridazinyl)methyl]-4-tritylthio-azetidin-2-one

The deprotected azetidinone (60) (Preparation 8(b), 0.85 g) was acetylated in the manner described in Preparation 6(c).

Chromatography (silica gel eluted with ethyl acetate) gave the title azetidinone (61) 0.85 g. $\nu_{max}$ ($CHCl_3$) 3400, 1775 (sh), 1770 $cm^{-1}$; $\delta$ppm ($CDCl_3$) 3.88 (1H, m); 4.20 (1H, s); 4.64 (1H, d, J=3Hz); 6.22 (1H, d, J=6Hz); 7.10-7.60 (17H, m); 9.17 (1H, m).

Preparation 8(d)

(3RS, 4SR) 3[Acetoxy-(3-pyridazinyl)methyl]-1-(1-p-nitrobenzyloxycarbonyl-1-triphenylphosphoranylidenemethyl)-4-tritylthio-azetidin-2-one

The azetidinone (61) (1.35 g) from Preparation 8(c) was reacted with p-nitrobenzylglyoxylate (0.68 g, 1.1 eq) as described in Preparation 6(d) to give the crude hydroxy ester as a foam, 2.0 g $\nu_{max}$ ($CHCl_3$) 1765 $cm^{-1}$.

A solution of the hydroxy ester (124 mg) in dry THF (5 ml) was cooled to -20°C and 2,6-lutidine (24 µl) added. A solution of thionyl chloride (14 µl) in dry THF (2 ml) was added dropwise with stirring and the reaction continued for 20 minutes at -15°C. Filtration and evaporation of the solvent gave a foam to which was added dry dioxan (1 ml), triphenylphosphine (133 mg, 3 eq) and 2,6-lutidine (29 µl, 1.5 eq). The resulting mixture was stirred at 45°C under an atmosphere of argon for 12 hours.

The reaction mixture was dissolved in ethyl acetate (40 ml) and washed with 1N HCl (5 ml), aqueous sodium bicarbonate solution and with brine. Drying ($MgSO_4$) and evaporation of the solvent gave a crude product which was chromatographed on silica gel eluting with ethyl acetate-n-hexane.

The title phosphorane (62) was obtained as a foam, 50 mg, $\nu_{max}$ ($CHCl_3$) 1755, 1625 to 1605 $cm^{-1}$.

Preparation 8(e)

(3RS, 4SR) Silver 3-[acetoxy(3-pyridazinyl)methyl]-1-(1-p-nitrobenzyloxycarbonyl-1-triphenylphosphoranylidene-methyl)azetidin-2-one-4-thiolate

The phosphorane (62) (0.80 g) from the Preparation 8(d) was dissolved in a mixture of methanol (5.4 ml) and dry MDC (2.0 ml). Pyridine (0.088 ml) was added followed by the dropwise addition of a solution of silver nitrate in methanol (0.15M; 6.7 ml).

This solution was stirred for 1.0 hour at ambient temperature and then decanted from the separated black solid. The decanted solution was concentrated to 50% volume, an equal volume of dry $Et_2O$ added, and chilled at 5°C for 0.5 hour. The resulting precipitated solid was separated by filtration, washed with ether and dried to give 0.42 g of the title thiolate (63) $\nu_{max}$ ($CHCl_3$) 1745, 1610 $cm^{-1}$.

Example 8(a)

(5RS) p-Nitrobenzyl 6(3-pyridazinyl)methylenepenem-3-carboxylate

The thiolate (63) (Preparation 8(e), 420 mg) was dissolved in dry MDC (10 ml) and cooled in an ice bath. 4-Dimethylaminopyridine (69 mg, 1.1 eq) and acetic formic anhydride (400 µl, 10 eq) were added followed by triethylaminehydrochloride (355 mg, 5 eq). The mixture was stirred 5 minutes at $0^oC$ when a precipitate formed. The ice bath was removed and stirring continued for 0.5 hour. The precipitate was removed by filtration and washed well with ethyl acetate. The filtrate was washed with 1N HCl, aqueous sodium bicarbonate solution and with brine. Drying ($MgSO_4$) gave an ethyl acetate solution which was diluted to a volume of 100 ml and heated at $40^oC$ under an atmosphere of argon for 10 minutes, followed by 1.0 hour to ambient temperature.

The ethyl acetate solution was then chilled to $-40^oC$ under an atmosphere of argon and DBU (250 mg) dissolved in ethyl acetate (10 ml) added. After stirring at $-40^oC$ for 30 minutes the ethyl acetate solution was washed with 5% citric acid (50 ml), saturated sodium bicarbonate solution (2 x 50 ml), and brine (50 ml). The dried ($MgSO_4$) organic layer was evaporated and the residue chromatographed on silica gel eluting with ethyl acetate to give the title penem (64) 70 mg as a yellow crystalline solid. $\lambda_{max}$ (EtOH) 260 nm ($\epsilon$m 19000), 310 nm ($\epsilon$m 7940). $\nu_{max}$ ($CHCl_3$) 1785, 1720 $cm^{-1}$; $\delta$ppm ($CDCl_3$) 5.30 (1H, d, J=13.5Hz); 5.47 (1H, d, J=13.5Hz); 6.74 (1H, d, J=1Hz) 7.12 (1H, d, J=1Hz); 7.41 (1H, s); 7.53-7.65 (4H, m); 8.23 (2H, m); 9.17 (1H, m).

Example 8(b)

(5RS) Sodium 6-(3-pyridazinyl)-methylenepenem-3-carboxylate

The penem ester (64) (Example 8(a); 70 mg) was hydrogenolysed and converted to the sodium salt as described in Example 5(b).

The title compound (65) was isolated as a yellow solid 25 mg, $\lambda_{max}$ ($H_2O$) 252 nm ($\epsilon$m 14330); $\nu_{max}$ (KBr) 1760, 1600 $cm^{-1}$; $\delta$ppm ($D_2O$) 6.64 (1H, s); 7.03 (1H, s); 7.25 (1H, s); 7.80-7.85 (2H, m); 9.08 (1H, m).

Preparation 9

Ethyl 5,6-dimethyl-1,2,4-triazine-3-carboxylate

(cf. D. Krass and W. Paudler, Synthesis 1974, 351).

The reaction of ethyl oxalamidrazonate (480 mg) with 2,3-butanedione (0.4 ml) in dry THF gave the title ester, 428 g as an oil, after chromatographic purification. The i.r. spectrum showed absorption at 1740 $cm^{-1}$.

Preparation 9(a)

(3RS, 4SR) N-t-Butyldimethylsilyl-3-(5,6-dimethyl-1,2,4-triazinyl-3-carbonyl)-4-tritylthioazetidin-2-one

Using the method described in Preparation 4(a), azetidinone (1) (459 mg) was condensed with ethyl 5,6-dimethyl-1,2,4-triazine-3-carboxylate (270 mg). Purification by column chromatography on silica gel, eluting with ethyl acetate/n-hexane gave the title compound

(66) as a foam, 230 mg; $\nu_{max}$ ($CHCl_3$) 1750, 1710 $cm^{-1}$; $\delta$($CDCl_3$) 0.82 (9H, s); 2.58 (3H, s); 2.72 (3H, s); 4.99 (1H, d, J=2Hz); 5.22 (1H, d, J=2Hz); 6.9-7.5 (15H, m); signal for $\underline{Me}_2Si$ obscured by TMS

Preparation 9(b)

(3RS, 4SR) N-t-Butyldimethylsilyl-3-[(5,6-dimethyl-1,2,4-triazin-3-yl)-hydroxymethyl]-4-tritylthioazetidin-2-one

The azetidinone-ketone (66) (1.85 g) from Preparation 9(a) was treated with sodium borohydride (145 mg) in the manner described in Preparation 4(b).

Chromatography of the crude product gave two isomers:- isomer I (67), 633 mg; $\nu_{max}$ ($CHCl_3$) 3200-3500, 1740 $cm^{-1}$; $\delta$($CDCl_3$) 0.9 (9H, s); 2.48 (3H, s); 2.60 (3H, s); 3.75-3.85 (1H, br); 3.90 (1H, dd, J=2,2Hz); 4.0-4.15 (ca. 1H, br); 4.65 (1H, d, J=2Hz); 7.1-7.6 (ca 15H, m);

isomer II (68), (more polar) white solid, 623 g, $\nu_{max}$ ($CHCl_3$) 3200-3600, 1740 $cm^{-1}$; $\delta$($CDCl_3$) 0.4 (3H, s); 0.56 (3H, s); 0.8 (9H, s); 3.3-3.6 (1H, br); 3.91 (1H, dd, J=9,2Hz); 4.36 (1H, d, J=2Hz); 4.45-4.65 (1H, br); 7.1-7.5 (15H, m).

Preparation 9(c)

(3RS, 4SR) 3-[Acetoxy-(5,6-dimethyl-1,2,4-triazin-3-yl) methyl]-4-tritylthio-azetidin-2-one

The azetidinone-alcohol, isomer II (68) (623 mg) from Preparation 9(b) was dissolved in dry methanol (15 ml) - methylene chloride (1.5 ml) and cooled to -30°C. A solution of potassium fluoride (81 mg) in methanol (2 ml)

was added dropwise with stirring. Over a period of 1 hour the temperature was allowed to rise to 0° and tlc showed no starting material. Dilution with brine, extraction with ethyl acetate, drying ($MgSO_4$) and evaporation gave crude desilylated azetidinone as a foam, 516 mg, ir $\nu_{max}$ ($CHCl_3$) 3200-3500, 3400 sharp, 1765 $cm^{-1}$. This azetidinone was used below without further purification.

The crude desilylated azetidinone (516 mg) was dissolved in $CH_2Cl_2$ (10 ml) and triethylamine (0.16 ml) and 4-dimethylaminopyridine (9 mg) added. The solution was cooled in ice and acetic anhydride (0.116 ml) added. After 50 min at 0° the solution was washed with 1N HCl, aqueous $NaHCO_3$ and with brine. Drying ($MgSO_4$) and evaporation gave a crude product which was purified by chromatography to give the title compound (69) as a foam, 488 mg; $\nu_{max}$ ($CHCl_3$) 3400, 1770 $cm^{-1}$; $\delta$($CDCl_3$) 2.1 (3H, s); 2.56 (3H, s); 2.72 (3H, s); 3.87 (1H, dd, J=4.3, 3Hz); 4.2 (1H, brs); 4.70 (1H, d, J=3Hz); 6.12 (1H, d, J=4.3Hz); 7.15-7.4 (15H, m).

## Preparation 9(d)

**(3RS, 4SR) 3-[Acetoxy-(5,6-dimethyl-1,2,4-triazin-3-yl) methyl]-1-(1-p-nitrobenzyloxycarbonyl-1-triphenylphosphoranylidenemethyl)-4-tritylthio-azetidin-2-one.**

The azetidinone-acetate (69) (514 mg) from Preparation 9(c) was condensed with p-nitrobenzyl glyoxylate using the procedure described in Preparation 5(e). Subsequent reaction of the resultant hydroxy-ester with thionyl chloride, again using the method of Preparation 5(e), gave a crude chloro-ester as a buff-coloured foam. Further reaction with excess triphenylphosphine in the presence of

2,6-lutidine (as described in Preparation 5(e) gave, after column chromatography, the title phosphorane (70) as an off white foam, 661 mg; $\nu_{max}$ ($CHCl_3$) 1750, 1600-1620 $cm^{-1}$.

Preparation 9(e)

(3RS, 4SR) Silver 3-[acetoxy-(5,6-dimethyl-1,2,4-triazin-3-yl)-methyl]-1-(1-p-nitrobenzyloxycarbonyl-1-triphenylphosphoranylidenemethyl)azetidin-2-one.

The phosphorane (70) (746 mg) from Preparation 9(d) was dissolved in methanol (10 ml)-methylene chloride (1 ml). Pyridine (0.08 ml) was added followed by a solution of silver nitrate in methanol (0.15M; 6.1 ml). After 40 minutes at room temperature the solution was cooled to $0^{o}$ and stirred for 1 hour. A small portion (50 mg) of the desired product was then isolated as on off white solid by filtration.

Evaporation of the above filtrate gave a gum which was triturated with several portions of ether to give more of the title compound (71) as a grey solid, 576 mg, $\nu_{max}$ ($CHCl_3$) 1745, 1600-1620 $cm^{-1}$.

Preparation 9(f)

(5RS, 6SR) p-Nitrobenzyl 6-[acetoxy-(5,6-dimethyl-1,2,4-triazin-3-yl)methyl]penem-3-carboxylate

The silver thiolate (71) (420 mg) from Preparation 9(e) was dissolved in dry methylene dichloride (10 ml) and cooled in an ice bath. Acetic formic anhydride (0.4 ml) and 4-dimethylaminopyridine (69 mg) were added followed by

triethylamine hydrochloride (370 mg). After 5 minutes of stirring a precipitate formed and the cooling bath was removed.

After 30 minutes the mixture was diluted with ethyl acetate and filtered. The filtrate was diluted to 100 ml with EtOAc and washed with cold water, 1N HCl, aqueous $NaHCO_3$ and with brine. The resulting organic solution was dried ($MgSO_4$) and then heated at 40° for 45 minutes.

Evaporation gave a gum which was purified by repeated chromatography on silica gel, ethyl acetate/n-hexane. The title compound (72) was obtained as a pale yellow solid 11 mg, $\lambda_{max}$ (EtOH) 261 ($\varepsilon$ 17100); 315 nm ($\varepsilon$ 8400); $\nu_{max}$ ($CHCl_3$) 1795, 1750, 1720 $cm^{-1}$; $\delta$($CDCl_3$) 2.19 (3H, s); 2.56 (3H, s); 2.71 (3H, s); 4.63-4.65 (1H, m); 5.27 (1H, d, J=13.5Hz); 5.42 (1H, d, J=13.5Hz); 6.02 (1H, d, J=2Hz); 6.38 (1H, d, J=3.7Hz); 7.31 (1H, d, J=1Hz); 7.57-7.61 (2H, m); 8.22-8.25 (2H, m).

A further 179 mg of the title compound (72) was also obtained, slightly contaminated with triphenylphosphine oxide but this was judged of sufficient purity for use in Example 9(a).

## Example 9(a)

### (5RS) p-Nitrobenzyl 6-[(5,6-dimethyl-1,2,4-triazin-3-yl) methylene]penem-3-carboxylate

The acetoxy-penem (72) (169 mg) from Preparation 9(f) was dissolved in dry methylene dichloride (10 ml) and cooled to -30° under argon. A solution of DBU (63 mg) in methylene dichloride (1 ml) was added dropwise with stirring.

After 30 minutes tlc showed no starting material and the solution was washed with 1N HCl, aqueous $NaHCO_3$ and with brine. Drying ($MgSO_4$) and evaporation gave a crude product which was purified by repeated chromatography on silica gel (Ethyl acetate/n-hexane and ethyl acetate/methylene dichloride). The product (73) was obtained as a yellowsolid, 93 mg, $\lambda_{max}$ (EtOH) 262 nm $\epsilon m$ 30,500; $\nu_{max}$ ($CHCl_3$) 1785, 1715 $cm^{-1}$; $\delta$($CDCl_3$) 2.60 (3H, s); 2.75 (3H, s); 5.31 (1H, d, J=13.5Hz); 5.47 (1H, d, J=13.5Hz); 6.66 (1H, d, J=1Hz); 7.34 (1H, d, J=1Hz); 7.39 (1H, s); 7.61-7.65 (2H, arom); 8.24-8.27 (2H, arom).

A sample of (73) crystallized from ethyl acetate containing a trace of n-hexane gave orange crystals, mp. 185-187$^{o}$ decomp.

## Example 9(b)

## (5RS) Sodium 6-[(5,6-dimethyl-1,2,4-triazin-3-yl)methylene] penem-3-carboxylate

The penem ester (73) (63 mg) from Example 9(a) was dissolved in dioxan (16 ml) and water (4 ml). Sodium bicarbonate solution (1% in water; 1.25 ml) was added followed by 5% Pd/C (90 mg). After hydrogenation at room temperature and 1 atmosphere pressure for 20 minutes tlc showed none of the starting material to be present.

The catalyst was removed by filtration and the filtrate was evaporated to dryness. The residue was redissolved in a small volume of water and passed through a column of Biogel P2, eluting with water. Fractions were combined on the basis of a uv $\lambda_{max}$ ca. 261 nm. Freeze

drying of the combined fractions gave the title compound (74) as a yellow freeze-dried solid, 27 mg, $\lambda_{max}$ ($H_2O$) 260 nm, $\varepsilon$m16,900; $\nu_{max}$ (KBr) 1760, 1600 $cm^{-1}$; $\delta$($D_2O$) 2.60 (3H, s); 2.65 (3H, s); 6.65 (1H, s); 7.06 (1H, s); 7.20 (1H, s).

Biological data

The following Table 1 summaries the antibacterial activity of selected compounds according to the invention (which are identified by compound numbers as given in the examples) against selected micro-organisms and, for comparison purposes, also gives the same data for amoxycillin. The data is given in the form of MIC values (minimum inhibitory concentration) in µg/ml.

Table 2 summaries the β-lactamase activity of the compounds listed in Table 1 when used in conjunction with amoxycillin against the same micro-organisms. The data is given in the form of the minimum inhibitory amount of amoxycillin in µg/ml when used in conjunction with 5 µg/ml of the respective compound according to the invention.

TABLE 1

Antibacterial Activity of Compounds Alone (MIC μg/ml)

| Compound No (see Examples) | P mirabilis C889 | E coli JT410 | C freundii Mantio | E aerogenes N1 |
|---|---|---|---|---|
| 29 | >100 | >100 | >100 | >100 |
| 39 | >512 | >512 | >512 | >512 |
| 58 | >512 | >512 | >512 | >512 |
| 65 | 256 | 256 | >512 | >512 |
| Amoxycillin* | >512 | 256 | >512 | 512 |

*Typical MIC's from a number of tests

TABLE 2

Amoxycillin MIC values (μg/ml) in the presence of compounds of this invention (5 μg/ml)

| Compound No (see Examples) | P mirabilis C889 | E coli JT410 | C freundii Mantio | E aerogenes N1 |
|---|---|---|---|---|
| 29 | 1 | 8 | 8 | 8 |
| 39 | 1 | 2 | 128 | 8 |
| 58 | 16 | 4 | 64 | 16 |
| 65 | 1 | 2 | 4 | 4 |

ep

Claims

1. A compound of the general formula I:

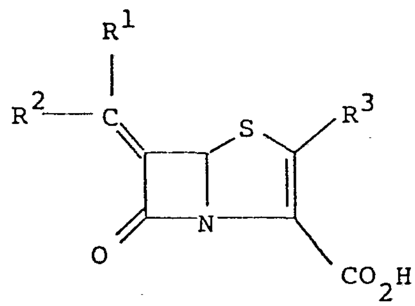

I

or a pharmaceutically acceptable salt or in vivo hydrolysable ester thereof

in which

one of $R^1$ and $R^2$ denotes hydrogen,

the other of $R^1$ and $R^2$ denotes an unsubstituted or substituted six-membered hetero-aromatic ring bonded through a carbon atom thereof and having from one to three nitrogen atoms as ring hetero-atoms, and

$R^3$ denotes hydrogen or an organic group.

2. A compound of the general formula IA

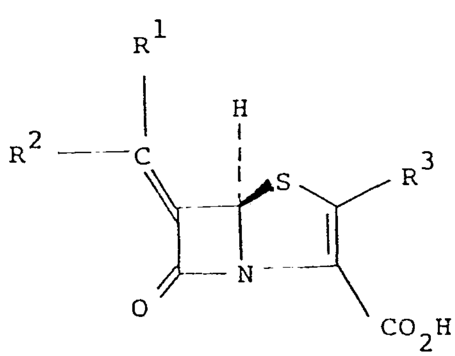

IA

or a pharmaceutically acceptable salt or in vivo hydrolysable ester thereof

in which $R^1$, $R^2$ and $R^3$ are defined as in claim 1.

3. A compound as claimed in claim 1 or claim 2, wherein $R^1$ denotes the hetero-aromatic group and $R^2$ denotes a hydrogen atom.

4. A compound as claimed in any one of claims 1 to 3, wherein the hetero-aromatic group is a pyridyl, pyrazinyl, pyrimidinyl, pyridazinyl, or triazinyl group.

5. A compound as claimed in any one of claims 1 to 4, wherein $R^3$ denotes hydrogen or a group of formula $-R^4$ or $-SR^4$, where $R^4$ denotes an unsubstituted or substituted $(C_{1-10})$ hydrocarbon or heterocyclyl group.

6. A compound selected from:

(5RS) 2-methyl-(Z)-6-(3-pyridylmethylene)penem-3-carboxylic acid;

(5RS) 2-ethylthio-6-(3-pyridylmethylene)penem-3-carboxylic acid;

(5RS) 6-(3-pyridylmethylene)penem-3-carboxylic acid;

(5RS) 6-pyrazinylmethylene-penem-3-carboxylic acid;

(5RS) 6-(4-pyrimidinylmethylene)penem-3-carboxylic acid;

(5RS) 6-(4-pyridylmethylene)penem-3-carboxylic acid;

(5RS) (Z) 6-(6-methoxy-3-pyridyl)methylene-penem-3-carboxylic acid;

(5RS) (E) 6-(6-methoxy-3-pyridyl)methylene-penem-3-carboxylic acid;

(5RS) 6-(3-pyridazinyl)methylene-penem-3-carboxylic acid; and

(5RS) 6-[(5,6-dimethyl-1,2,4-triazin-3-yl)methylene] penem-3-carboxylic acid;

or a pharmaceutically acceptable salt or <u>in-vivo</u> hydrolysable ester thereof.

7. A process for the preparation of a compound of the general formula I as defined in claim 1, or a pharmaceutically acceptable salt or *in vivo* hydrolysable ester thereof, which comprises eliminating the elements of a compound of the general formula II:

H-X II

from a penem or penem intermediate of the general part-formula III:

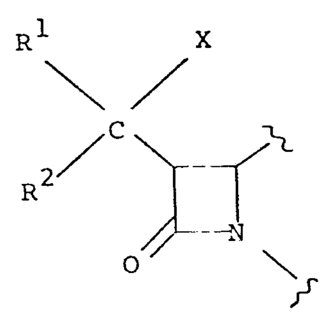

III

in which formulae

$R^1$ and $R^2$ are defined as in claim 1, and

X denotes a hydroxy group or a leaving group,

to give a compound of the general part-formula IV:

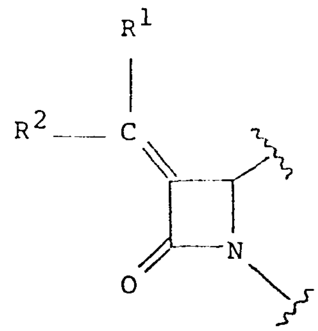

IV

in which $R^1$ and $R^2$ are defined as in claim 1,

and, if the resulting compound of the general formula IV is a penem intermediate, converting it into a penem of the general formula I or a pharmaceutically acceptable salt or *in vivo* hydrolysable ester thereof.

8. A process for the preparation of a compound of the general formula I given in claim 1, in which $R^1$ and $R^2$ are defined as in claim 1 and $R^3$ denotes a group of the formula $-SR^4$ (in which $R^4$ is defined as in claim 5), or a pharmaceutically acceptable salt or *in vivo* hydrolysable ester thereof, which comprises reacting a compound of the general formula XIII:

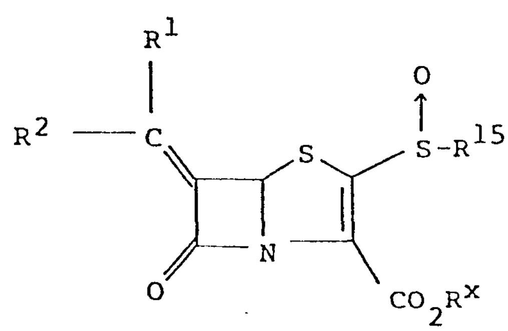

XIII

in which

$R^1$ and $R^2$ are defined as in claim 1,

$R^{15}$ denotes an organic radical different from the group $R^4$, and

$R^x$ denotes hydrogen or a carboxyl-blocking group,

with a thiol of the general formula XI:

$$R^4\text{-SH} \qquad XI$$

in which $R^4$ is defined as in claim 5,

or a reactive derivative thereof;

and thereafter if necessary or desired:

(a) removing any carboxyl-blocking group $R^x$,

and/or

(b) converting the product into the free acid or into a pharmaceutically acceptable salt or *in vivo* hydrolysable ester.

9. A compound of the general formula IIIC:

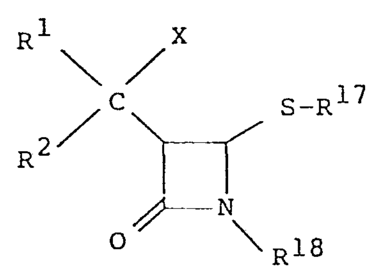

IIIC

in which

$R^1$ and $R^2$ are defined as in claim 1,

$R^{17}$ denotes $(C_{1-6})$ alkyl, aryl, aryl$(C_{1-6})$alkyl, $(C_{1-6})$alkylthio, arylthio, hetero-aromatic-thio, acyl (for example, $(C_{1-6})$alkylcarbonyl, especially acetyl), $(C_{2-6})$alkenyl (especially vinyl), or aryl$(C_{2-6})$alkenyl, all of which may optionally be substituted,

$R^{18}$ denotes hydrogen or an N-protecting group, and

X denotes a hydroxy group or a leaving group.

10. A compound of the general formula IVC:

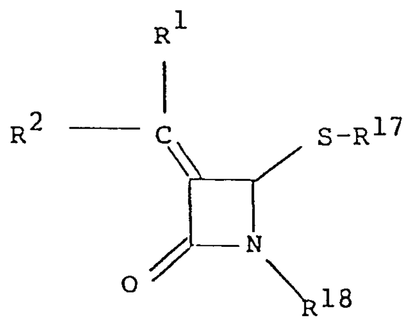

IVC

in which, $R^1$ and $R^2$ are defined as in claim 1 and $R^{17}$ and $R^{18}$ are defined as in claim 11.

11. A compound of the general formula IIID:

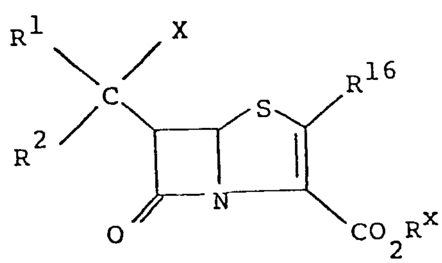

IIID

in which

$R^1$ and $R^2$ are defined as in claim 1.

$R^{16}$ denotes hydrogen or an organic group,

$R^x$ denotes hydrogen or a carboxyl-blocking group, and

X denotes a hydroxy group or a leaving group.

12. A compound of the general formula IVE:

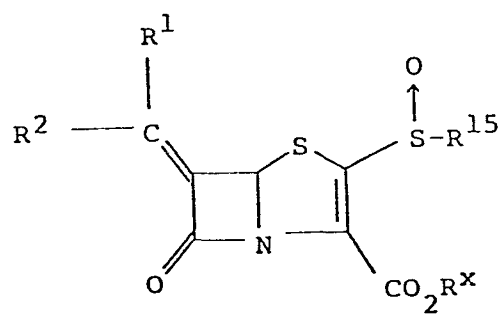

IVE

in which

$R^1$ and $R^2$ are defined as in claim 1,

$R^{15}$ denotes an organic group, and

$R^x$ denotes hydrogen or a carboxyl-blocking group.

13. A pharmaceutical composition comprising a compound as claimed in any one of claims 1 to 6 and a pharmaceutically acceptable carrier.

14. A pharmaceutical composition as claimed in claim 13, which additionally comprises a penicillin, cephalosporin or other β-lactam antibiotic.

15. A compound as claimed in any one of claims 1 to 6 for use as a therapeutically active substance.

16. A compound as claimed in any one of claims 1 to 6 for use in the treatment of a bacterial infection.

17. A compound as claimed in any one of claims 1 to 6 for use in the treatment of a bacterial infection in admixture or conjunction with a penicillin, cephalosporin or other β-lactam antibiotic.

18. A compound as claimed in any one of claims 1 to 6 for use as a β-lactamase inhibitor.

European Patent Office

# EUROPEAN SEARCH REPORT

Application number

EP 85 10 0520

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
| --- | --- | --- | --- |
| D,A | EP-A-0 041 768 (BEECHAM)<br>* Claims *<br>----- | 1,7-18 | C 07 D 499/00<br>C 07 D 401/06<br>C 07 D 403/06<br>C 07 F 7/10<br>A 61 K 31/43 //<br>C 07 F 9/65<br>C 07 D 239/28<br>C 07 D 253/06 |
| | | | TECHNICAL FIELDS SEARCHED (Int. Cl.4) |
| | | | C 07 D 499/00<br>A 61 K 31/00<br>C 07 D 401/00<br>C 07 D 403/00<br>C 07 D 205/00 |

The present search report has been drawn up for all claims

| Place of search | Date of completion of the search | Examiner |
| --- | --- | --- |
| THE HAGUE | 11-04-1985 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82